# EUROPÄISCHE PATENTSCHRIFT

(11) **EP 2 054 378 B1**
(45) Veröffentlichungstag und Bekanntmachung des Hinweises auf die Patenterteilung: **17.02.2016**
(21) Anmeldenummer: 07803111.9
(22) Anmeldetag: 31.08.2007
(51) Int. Cl.: C07C 253/00, C07C 255/16

(54) **VERFAHREN ZUR HERSTELLUNG VON CYANHYDRINEN SOWIE DEREN VERWENDUNG BEI DER HERSTELLUNG VON METHACRYLSÄUREALKYLESTERN**
PROCESS FOR PREPARING CYANOHYDRINS AND THEIR USE IN THE PREPARATION OF ALKYL ESTERS OF METHACRYLIC ACID
PROCEDE DE PRODUCTION DE CYANHYDRINES ET LEUR UTILISATION POUR PRODUIRE DES ESTERS ALKYLIQUES DE L'ACIDE METHACRYLIQUE

(30) Priorität: 08.12.2006 DE 102006058249
(43) Veröffentlichungstag der Anmeldung: 06.05.2009
(73) Patentinhaber: Evonik Röhm GmbH, 64293 Darmstadt (DE)
(72) Erfinder: GROPP, Udo, 83093 Bad Endorf (DE); WEBER, Robert, 64665 Alsbach-hähnlein (DE); SCHÄFER, Thomas, 64572 Büttelborn (DE); PERL, Andreas, 67240 Bobenheim-roxheim (DE); SING, Rudolf, 67551 Worms (DE); MERTZ, Thomas, 64625 Bensheim (DE)
(86) Internationale Anmeldenummer: PCT/EP2007/059112
(87) Internationale Veröffentlichungsnummer: WO 2008/068065

(56) Entgegenhaltungen:
- EP-A- 0 022 138
- EP-A- 1 666 451
- WO-A-00/64861
- WO-A-02/051534
- WO-A-2005/028097
- DE-A1- 2 927 979
- US-A- 6 030 534
- US-A1- 2003 147 791
- US-A1- 2006 165 566
- US-B2- 7 014 821

## Beschreibung

Die vorliegende Erfindung betrifft ein Verfahren zur Herstellung von Acetoncyanhydrin sowie ein Verfahren zur Herstellung von Methacrylsäurealkylestern, bei dem ein Acetoncyanhydrin wie es gemäß der vorliegenden Erfindung hergestellt werden kann eingesetzt wird.

Die Herstellung von Methacrylsäurealkylester umfasst häufig mindestens einen Verfahrensschritt, bei dem Acetoncyanhydrin als Ausgangsprodukt dient. Cyanhydrine sind Verbindungen, die bei erhöhter Temperatur häufig thermischen Zerfallsprozessen unterliegen. Als Hauptzerfallsprodukte bilden sich in diesem Fall häufig wieder Blausäure und Aceton, oft werden jedoch auch noch weitere Nebenprodukte festgestellt.

Der Zerfall von Cyanhydrinen erfolgt unter den geeigneten Umständen häufig bei Temperaturen von mehr als etwa 70 °C. Der Zerfall von Cyanhydrinen bei Reaktionen, in denen das Cyanhydrin ein Zwischenprodukt oder ein Reaktand für weiterführende Reaktionen darstellt, beispielsweise bei der Herstellung von Methacrylsäurealkylestern, wirkt sich ein derartiger thermischer Zerfall von Cyanhydrinen direkt im Rahmen einer Ausbeuteverminderung bei der Gesamtreaktion aus. Aus diesem Grund haben es sich viele aus dem Stand der Technik bekannte Druckschriften zur Aufgabe gemacht, eine entsprechende thermische Zersetzung von Cyanhydrinen bei ihrer Herstellung möglichst weitgehend zu verhindern.

Die US 2006/0111586 A1 betrifft ein Verfahren zur Minimierung der Zersetzung von Cyanhydrinen bei der Weiterverarbeitung in einem exothermen Reaktionsprozess, bei dem Cyanhydrine in einem Schlaufenreaktor mit mindestens einer Reaktionszone und einer Mischvorrichtung umgesetzt werden, wobei dem Reaktionsgemisch eine Wärmemenge entzogen wird, welche der im Rahmen der exothermen Reaktion produzierten Wärme äquivalent ist. Gemäß weiteren Ausführungen der Druckschrift wird insbesondere die Bildung von sogenannten "hot-spots" dadurch unterbunden, dass eine äußerst komplexe Zugabe der Reaktanden vorgeschlagen wird. Problematisch bei dem vorgeschlagenen Verfahren wirkt sich jedoch aus, dass die dargestellten Maßnahmen ein hohes Maß an intelligenter Steuerungskapazität erfordern und demnach äußerst fehleranfällig sein können. Darüber hinaus wird auf eine entsprechende Problematik bei der Herstellung von Cyanhydrinen nicht eingegangen.

Die WO00/64861 offenbart ein kontinuierliches Verfahren zur Herstellung von Acetoncyanhydrin aus Cyanwasserstoff und Aceton. Die aus dem Reaktor abgeführte Gasphase wird in einem nachgeschalteten Gaswäscher behandelt und die erhaltene Waschphase in den Gas-Flüssigreaktor zurückgeführt. Durch den Einsatz dieses als Reaktionskolonne betriebenen Gaswäschers kann insbesondere ein Rohgas aus dem BMA- oder Andrussow-Prozess eingesetzt werden.

Der vorliegenden Erfindung lag daher die Aufgabe zugrunde, ein Verfahren zur Herstellung von Acetoncyanhydrin zur Verfügung zu stellen, das sich im Rahmen eines übergeordneten Verfahrens zur Herstellung von Methacrylsäurealkylestern einsetzen lässt. Der Erfindung lag weiterhin die Aufgabe zugrunde, ein Verfahren zur Herstellung von Acetoncyanhydrin zur Verfügung zu stellen, das eine möglichst einfache Kontrolle der Reaktionsbedingungen erlaubt und möglichst wenig Steuerungsaufwand benötigt. Darüber hinaus lag der vorliegenden Erfindung die Aufgabe zugrunde, ein Verfahren zur Herstellung von Acetoncyanhydrin zur Verfügung zu stellen, dass bei gleichzeitig einfacher Verfahrensführung die thermische Zersetzung der Cyanhydrine möglichst minimiert.

Diese und weitere der Erfindung zugrunde liegende Aufgaben, wie sie sich dem Fachmann bei Kenntnis des Standes der Technik aus dem vorliegenden Text ergeben, werden durch Verfahren gelöst, wie sie nachfolgend näher beschrieben werden.

### Zusammenfassung der Erfindung

Die Erfindung betrifft ein Verfahren zur Herstellung von Acetoncyanhydrin, bei dem ein Reaktionsgemisch enthaltend Aceton und Blausäure in Gegenwart eines Katalysators in einem Schlaufenreaktor unter Bildung von Acetoncyanhydrin als Reaktionsprodukt zur Reaktion gebracht werden, wobei der Schlaufenreaktor mindestens eine Vorrichtung zum Kühlen des Reaktionsgemischs, mindestens eine Pumpe, mindestens eine Vorrichtung zum Vermischen des Reaktionsgemischs und jeweils mindestens eine Zufuhröffnung zum Zuführen von Blausäure, Aceton und Katalysator aufweist, wobei die Zufuhr des Katalysators stromabwärts von der Zufuhr von Aceton und Blausäure erfolgt.

Die Zufuhr des Katalysators erfolgt dabei beispielsweise an einer Stelle des Schlaufenreaktors, die näher an einer Eintrittsöffnung des Reaktionsgemischs in mindestens eine Vorrichtung zum Vermischen des Reaktionsgemischs liegt als beide Zufuhröffnungen zum Zuführen von Blausäure oder Aceton. Dabei kann für die Zufuhr des Katalysators gelten, dass sie an einer Stelle des Schlaufenreaktors erfolgt, an welcher der Weg des Reaktionsgemischs vor Eintritt in eine Eintrittsöffnung in mindestens eine Vorrichtung zum Vermischen des Reaktionsgemischs höchstens 30 % des Wegs beträgt, welchen das Reaktionsgemisch nach der Zufuhr von Blausäure und Aceton vor Eintritt in eine Eintrittsöffnung in mindestens eine Vorrichtung zum Vermischen des Reaktionsgemischs zurücklegt.

Die Verweildauer des Katalysators im Reaktionsgemisch zwischen Zufuhr und Vermischung in mindestens einer Vorrichtung zum Vermischen des Reaktionsgemischs beträgt beispielsweise höchstens etwa 10 s.

Die Zufuhr des Katalysators kann grundsätzlich kontinuierlich oder intermittierend erfolgen. Die Temperatur des Reaktionsgemischs im Schlaufenreaktor beträgt beispielsweise etwa 11 bis etwa 70 °C.

Im Rahmen einer weiteren Ausführungsform der Erfindung wird als Katalysator eine anorganische oder eine organische Base eingesetzt, beispielsweise ein organisches Amin oder ein Gemisch aus zwei oder mehr organischen Aminen.

Dem Schlaufenreaktor wird vorzugsweise kontinuierlich oder intermittierend Reaktionsprodukt entzogen, wobei das Reaktionsprodukt nach Verlassen des Schlaufenreaktors in einem oder mehreren Wärmetauschern auf eine Temperatur von etwa 0 bis etwa 10 °C gekühlt und im Rahmen einer weiteren Ausführungsform der Erfindung mit einer Säure, insbesondere mit Schwefelsäure, versetzt wird. Es hat sich in einigen Fällen als vorteilhaft erwiesen, wenn dabei Schwefelsäure mit einem Wassergehalt von 0,1 bis 8 Gew.-% eingesetzt wird.

Dem Schlaufenreaktor kann im Rahmen des erfindungsgemäßen Verfahrens beispielsweise an mindestens einer Stelle eine Probenmenge des Reaktionsgemischs entnommen werden, wobei die Probenmenge während oder nach der Entnahme spektroskopisch untersucht wird. Die Probennahme kann dabei kontinuierlich oder intermittierend erfolgen und die Probe kann dem Schlaufenreaktor nach der Messung wieder zugeführt werden.

Die Reaktion wird beispielsweise bei einem pH-Wert von etwa 6,5 bis etwa 8 durchgeführt.

Die Erfindung betrifft auch ein Verfahren zur Herstellung von Acetoncyanhydrin, bei dem ein Reaktionsgemisch enthaltend Aceton und Blausäure in Gegenwart eines Katalysators in einem Schlaufenreaktor unter Bildung von Acetoncyanhydrin als Reaktionsprodukt zur Reaktion gebracht werden und bei dem der Schlaufenreaktor mindestens eine Vorrichtung zum Kühlen des Reaktionsgemischs, mindestens eine Pumpe, mindestens eine Vorrichtung zum Vermischen des Reaktionsgemischs und jeweils mindestens eine Zufuhröffnung zum Zuführen von Blausäure, Aceton und Katalysator aufweist, wobei dem Schlaufenreaktor kontinuierlich oder intermittierend Reaktionsprodukt entzogen wird, das Reaktionsprodukt nach Verlassen des Schlaufenreaktors in einem oder mehreren Wärmetauschern auf eine Temperatur von 0 bis 10 °C gekühlt und mit Schwefelsäure mit einen Wassergehalt von 0,1 bis 8 Gew.-% versetzt wird. Die Zufuhr des Katalysators erfolgt dabei stromabwärts von der Zufuhr von Aceton und Blausäure.

Die Zufuhr des Katalysators kann dabei erfindungsgemäß ebenfalls an einer Stelle des Schlaufenreaktors erfolgen, die näher an einer Eintrittsöffnung des Reaktionsgemischs in mindestens eine Vorrichtung zum Vermischen des Reaktionsgemischs liegt als beide Zufuhröffnungen zum Zuführen von Blausäure oder Aceton, beispielsweise an einer Stelle des Schlaufenreaktors, an welcher der Weg des Reaktionsgemischs vor Eintritt in eine Eintrittsöffnung des Reaktionsgemischs in mindestens eine Vorrichtung zum Vermischen des Reaktionsgemischs höchstens 30 % des Wegs beträgt, welchen das Reaktionsgemisch nach der Zufuhr von Blausäure und Aceton vor Eintritt in eine Eintrittsöffnung des Reaktionsgemischs in mindestens eine Vorrichtung zum Vermischen des Reaktionsgemischs zurücklegt.

Die Verweildauer des Katalysators im Reaktionsgemisch zwischen Zufuhr und Vermischung in mindestens einer Vorrichtung zum Vermischen des Reaktionsgemischs beträgt auch in diesem Fall im Rahmen einer bevorzugten Ausführungsform der Erfindung höchstens etwa 10 s.

Die Zufuhr des Katalysators kann kontinuierlich oder intermittierend erfolgen, die Temperatur des Reaktionsgemischs im Schlaufenreaktor beträgt beispielsweise etwa 11 bis etwa 70 °C, bei der Reaktion kann als Katalysator eine anorganische oder eine organische Base eingesetzt werden, beispielsweise ein organisches Amin oder ein Gemisch aus zwei oder mehr organischen Aminen.

Dem Schlaufenreaktor kann erfindungsgemäß an mindestens einer Stelle eine Probenmenge des Reaktionsgemischs entnommen und die Probenmenge spektroskopisch untersucht werden. Die Probennahme kann kontinuierlich oder intermittierend erfolgen und die Probe kann dem Schlaufenreaktor nach der Messung wieder zugeführt werden. Die Reaktion wird beispielsweise bei einem pH-Wert von etwa 6,5 bis etwa 8 durchgeführt.

### Detaillierte Beschreibung der Erfindung

Gemäß der vorliegenden Erfindung wird das Reaktionsgemisch aus Aceton und Blausäure in einem Schlaufenreaktor zur Reaktion gebracht. Es ist erfindungsgemäß ebenso möglich und vorgesehen, dass anstatt eines einzigen Schlaufenreaktors eine Kaskade von zwei oder mehr Schlaufenreaktionen, beispielsweise drei, vier oder fünf Schlaufenreaktoren im Rahmen des Herstellungsverfahrens eingesetzt werden können. Die Schlaufenreaktoren können dabei beispielsweise seriell oder parallel angeordnet sein.

Ein im Rahmen der vorliegenden Erfindung einsetzbarer Schlaufenreaktor weist mindestens zwei Zufuhröffnungen zur Zufuhr von an der Reaktion beteiligten bzw. für die Reaktion erforderlichen Stoffen auf. Zu den im Rahmen der Reaktion erforderlichen Stoffen zählen gemäß dem vorliegenden Text auch solche Stoffe, die im Rahmen der Reaktion nicht notwendigerweise verbraucht werden, sondern für die Steuerung der Reaktionsgeschwindigkeit geeignet sind. Zu diesen Stoffen zählen beispielsweise Katalysatoren.

Bei der Umsetzung handelt es sich um eine exotherme Reaktion. Um einer Zersetzung des im Rahmen dieser Reaktion gebildeten Acetoncyanhydrin entgegenzuwirken, wird üblicherweise die Reaktionswärme durch eine geeignete Vorrichtung abgeführt. Die Umsetzung kann dabei grundsätzlich als Batchprozess oder als kontinuierliches Verfahren geführt werden, sofern eine kontinuierliche Fahrweise bevorzugt ist, wird die Umsetzung häufig in einem Schlaufenreaktor, der entsprechend eingerichtet ist, durchgeführt.

Ein Hauptmerkmal einer in hohen Ausbeuten zum gewünschten Produkt führenden Fahrweise liegt oft darin, dass bei ausreichender Reaktionszeit das Reaktionsprodukt gekühlt wird und das Reaktionsgleichgewicht in Richtung des Reaktionsproduktes verschoben wird. Darüber hinaus wird das Reaktionsprodukt zum Vorteil der Gesamtausbeute häufig mit einem entsprechenden Stabilisator versetzt, um einer Zersetzung bei der späteren Aufarbeitung in die Ausgangsstoffe vorzubeugen.

Die Vermischung der Reaktionspartner Aceton und Blausäure kann grundsätzlich auf im Wesentlichen beliebige Weise erfolgen. Die Art der Vermischung hängt insbesondere davon ab, ob eine diskrete Fahrweise, beispielsweise im Batchreaktor, oder eine kontinuierliche Fahrweise, im Schlaufenreaktor, gewählt wird.

Grundsätzlich kann es vorteilhaft sein, wenn das Aceton in die Reaktion über einen Vorlagebehälter eingespeist wird, der über einen Waschturm verfügt. Entlüftungsleitungen, die Aceton und Blausäure enthaltende Abluft führen, können so beispielsweise durch diesen Vorlagebehälter geführt werden. Im Waschturm, der an den Vorlagebehälter angeschlossen ist, kann die aus dem Vorlagebehälter entweichende Abluft mit Aceton gewaschen werden, wodurch Blausäure aus der Abluft entfernt und in den Prozess rückgeführt werden. Hierzu wird beispielsweise ein Teil der vom Vorlagebehälter in die Reaktion eingeführten Acetonmenge im Teilstrom über einen Kühler, vorzugsweise über einen Sole-Kühler, in den Kopf des Waschturms geführt und so das gewünschte Ergebnis erzielt.

Je nach Umfang der zu produzierenden Menge an Endprodukten kann es vorteilhaft sein, das Aceton aus mehr als nur einem Vorlagebehälter der Reaktion zuzuführen. Dabei kann jeder der zwei oder mehr Vorlagebehälter einen entsprechenden Waschturm tragen. Es ist jedoch in vielen Fällen ausreichend, wenn nur einer der Vorlagebehälter mit einem entsprechenden Waschturm ausgerüstet ist. In diesem Fall ist es jedoch oft sinnvoll, wenn entsprechende Abluft führende Leitungen, die Aceton und Blausäure transportieren können, über diesen Behälter bzw. über diesen Waschturm geführt werden.

Die Temperatur des Acetons im Vorlagebehälter kann grundsätzlich innerhalb eines im Wesentlichen beliebigen Bereiches liegen, insofern das Aceton sich bei der entsprechenden Temperatur im flüssigen Zustand befindet. Vorteilhafterweise beträgt die Temperatur im Vorlagebehälter jedoch etwa 0 bis etwa 20° C.

Im Waschturm wird das zum Waschen eingesetzte Aceton über einem entsprechenden Kühler, beispielsweise über einen Plattenkühler mit Sole auf eine Temperatur von etwa 0 bis etwa 10° C gekühlt. Die Temperatur des Acetons beim Eintritt in den Waschturm beträgt daher vorzugsweise beispielsweise etwa 2 bis etwa 6 °C.

Die im Rahmen der Reaktion benötigte Blausäure kann entweder in flüssiger oder in gasförmiger Form in den Reaktor eingeführt werden. Es kann sich dabei beispielsweise um Rohgas aus dem BMA- oder aus dem Andrussow-Prozess handeln.

Der Cyanwasserstoff kann beispielsweise verflüssigt werden, beispielsweise durch den Einsatz einer entsprechenden Kühlsole. Anstelle von verflüssigter Blausäure kann Kokereigas eingesetzt werden. So werden beispielsweise Cyanwasserstoff enthaltende Kokereigase nach einer Wäsche mit Pottasche kontinuierlich im Gegenstrom mit Aceton, das 10 % Wasser enthält, gewaschen und die Reaktion zu Acetoncyanhydrin kann in Gegenwart eines basischen Katalysators in zwei hintereinander geschalteten Gaswaschkolonnen durchgeführt werden.

Im Rahmen einer weiteren Ausführungsform kann ein Cyanwasserstoff und inerte Gase enthaltendes Gasgemisch, insbesondere ein Rohgas aus dem BMA- oder aus dem Andrussow-Prozess mit Aceton in Gegenwart eines basischen Katalysators und Acetoncyanhydrin in einem Gas-Flüssig-Reaktor umgesetzt werden.

Im Rahmen des hier beschriebenen Verfahrens wird vorzugsweise ein BMA-Rohgas oder ein Andrussow-Rohgas eingesetzt. Dass aus den oben genannten üblichen Verfahren zur Herstellung von Cyanwasserstoff resultierende Gasgemisch kann als solches oder nach einer Säurewäsche verwendet werden. Das Rohgas aus dem BMA-Prozess, bei welchem aus Methan und Ammoniak im Wesentlichen Blausäure und Wasserstoff gebildet werden, enthält typischerweise 22,9 Vol.-% HCN, 71,8 Vol.-% H₂, 2,5 Vol.-% NH3, 1,1 Vol.-% N₂, 1,7 Vol.-% CH₄. Im bekannten Andrussow-Prozess werden aus Methan und Ammoniak und Luftsauerstoff Blausäure und Wasser gebildet. Das Rohgas des Andrussow-Prozesses enthält beim Einsatz von Sauerstoff als Sauerstoffquelle typischerweise 8 Vol.-% HCN, 22 Vol.-% H₂, 46,5 Vol.-% N₂, 15 Vol.-% H₂O, 5 Vol.-% CO, 2,5 Vol.-% NH3 und je 0,5 Vol.-% CH4 und CO₂.

Beim Einsatz eines nicht säuregewaschenen Rohgases aus dem BMA-oder Andrussow-Prozess wirkt der im Rohgas enthaltene Ammoniak häufig als Katalysator für die Reaktion. Da der im Rohgas enthaltene Ammoniak häufig die als Katalysator erforderliche Menge übersteigt und deshalb zu hohen Verlusten an zur Stabilisierung eingesetzter Schwefelsäure führen kann, wird ein derartiges Rohgas oft einer Säurewäsche unterzogen, um Ammoniak daraus zu eliminieren. Beim Einsatz eines solchen Säure gewaschenen Rohgases muss dann allerdings ein geeigneter basischer Katalysator dem Reaktor in katalytischer Menge zugesetzt werden. Im Prinzip können dabei bekannte anorganische oder organische basische Verbindungen als Katalysator fungieren.

Cyanwasserstoff in gasförmiger oder in flüssiger Form bzw. ein Cyanwasserstoff enthaltendes Gasgemisch und Aceton werden im Rahmen der kontinuierlichen Fahrweise fortlaufend einem Schlaufenreaktor zugeführt. Der Schlaufenreaktor umfasst dabei mindestens eine Möglichkeit zum Zuführen von Aceton oder zwei oder mehr solcher Möglichkeiten, mindestens eine Möglichkeit zur Zuführung von flüssiger oder gasförmiger Blausäure, oder zwei oder mehr solcher Möglichkeiten sowie mindestens eine Möglichkeit zum Zuführen eines Katalysators.

Ein im Rahmen des beschriebenen Verfahrenselementes einsetzbarer Schlaufenreaktor weist darüber hinaus noch mindestens eine Pumpe, oder zwei oder mehr Pumpen, und mindestens eine Mischvorrichtung zur Durchmischung der Reaktionsgemischs , oder zwei oder mehr solcher Mischvorrichtungen auf. Als Pumpe eignen sich grundsätzlich alle Pumpen, die geeignet sind, den Umlauf des Reaktionsgemisches im Schlaufenreaktor zu gewährleisten.

Die Zugabe des Katalysators erfolgt vorzugsweise im Schlaufenreaktor nach der Pumpe und vor einem im Schlaufenreaktor vorhandenen Mischelement. Katalysatoren werden im Rahmen der beschriebenen Reaktion beispielsweise in einer solchen Menge eingesetzt, dass die Gesamtreaktion bei einem pH-Wert von maximal 8, insbesondere maximal etwa 7,5 oder etwa 7 gefahren wird. Es kann bevorzugt sein, wenn der pH-Wert bei der Reaktion sich innerhalb eines Rahmens von etwa 6, 5 bis etwa 7, 5, beispielsweise etwa 6,8 bis etwa 7,2 bewegt.

Wenn im Rahmen des beschriebenen Verfahrens eine kontinuierliche Fahrweise in einem Schlaufenreaktor als Betriebsart gewählt wird, so kann es zweckmäßig sein, den Zustand des Reaktionsgemisches durch punktuelle oder fortlaufende Analysen zu untersuchen. Dies bietet den Vorteil, dass gegebenenfalls auch auf Zustandsänderungen im Reaktionsgemisch schnell reagiert werden kann. Darüber hinaus lassen sich so beispielsweise die Reaktionspartner möglichst genau dosieren, um Ausbeuteverluste zu minimieren.

Eine entsprechende Analytik kann beispielsweise durch Probennahme in der Reaktorschlaufe erfolgen. Geeignete Analyseverfahren sind beispielsweise pH-Messung, Messung der Wärmetönung oder Messung der Zusammensetzung des Reaktionsgemisches durch geeignete spektroskopische Verfahren.

Insbesondere im Rahmen der Umsatzkontrolle, Qualitätsaspekten und Sicherheit hat es sich häufig bewährt, den Umsatz im Reaktionsgemisch über die aus dem Reaktionsgemisch abgeführte Wärme zu bestimmen und mit der theoretisch frei werdenden Wärme zu vergleichen.

Die eigentliche Reaktion kann bei geeigneter Wahl des Schlaufenreaktors grundsätzlich in den innerhalb des Schlaufenreaktors angeordneten Rohrsystemen erfolgen. Da die Reaktion jedoch exotherm ist, sollte, um Ausbeuteverlust zu vermeiden, auf eine ausreichende Kühlung bzw. auf eine ausreichende Abfuhr der Reaktionswärme geachtet werden. Es hat sich häufig als vorteilhaft erwiesen, wenn die Reaktion innerhalb eines Wärmetauschers, vorzugsweise innerhalb eines Rohrbündelwärmetauschers abläuft. Je nach zu produzierender Produktmenge kann die Kapazität eines entsprechenden Wärmetauschers unterschiedlich gewählt werden. Für großtechnische Verfahren haben sich insbesondere Wärmetauscher mit einem Volumen von etwa 10 bis etwa 40 m³ als besonders geeignet erwiesen. Bei den bevorzugt eingesetzten Rohrbündelwärmetauschern handelt es sich um Wärmetauscher die in einen flüssigkeitsdurchströmten Mantel ein flüssigkeitsdurchströmtes Rohrbündel aufweisen. Je nach Rohrdurchmesser, Packungsdichte, etc. kann der Wärmeübergang zwischen den beiden Flüssigkeiten entsprechend eingestellt werden. Es ist im Rahmend es beschriebenen Verfahrens grundsätzlich möglich, die Reaktion dahingehend zu führen, dass das Reaktionsgemisch durch den Wärmetauscher im Rohrbündel selbst gefahren wird und die Reaktion innerhalb des Rohrbündels stattfindet, wobei die Wärme aus dem Rohrbündel in die Mantelflüssigkeit abgeführt wird.

Es hat sich jedoch ebenfalls als praktikabel und in vielen Fällen als sinnvoll erwiesen, das Reaktionsgemisch durch den Mantel des Wärmetauschers zu führen, während die zur Kühlung eingesetzte Flüssigkeit innerhalb des Rohrbündels zirkuliert. Dabei hat es sich in vielen Fällen als vorteilhaft erwiesen, wenn die Reaktionsmischung im Mantel zur Erzielung einer höheren Verweildauer und einer besseren Durchmischung über Strömungswiderstände, vorzugsweise Umlenkbleche, verteilt wird.

Das Verhältnis von Mantelvolumen zum Volumen des Rohrbündels kann dabei, je nach Auslegung des Reaktors, etwa 10 zu 1 bis etwa 1 zu 10 betragen, vorzugsweise ist das Volumen des Mantels größer als das Volumen des Rohrbündels (bezogen auf den Inhalt der Rohre).

Die Wärmeabfuhr aus dem Reaktor wird mit einem entsprechenden Kühlmittel, beispielsweise mit Wasser, so eingestellt, dass die Reaktionstemperatur innerhalb eines Korridors etwa 25 bis etwa 45 °C, insbesondere bei etwa 30 bis etwa 38, insbesondere bei etwa 33 bis etwa 35 °C liegt.

Aus dem Schlaufenreaktor wird kontinuierlich ein Produkt abgeführt. Das Produkt weist eine Temperatur im Rahmen der oben genannten Reaktionstemperaturen, beispielsweise eine Temperatur von etwa 35 °C auf. Das Produkt wird über einen oder mehrere Wärmetauscher, insbesondere über einen oder mehrere Plattenwärmetauscher gekühlt. Dabei kommt beispielsweise eine Solekühlung zum Einsatz. Die Temperatur des Produktes nach Kühlung soll etwa 0 bis 10, insbesondere 1 bis etwa 5 °C betragen. Das Produkt wird vorzugsweise in einen Lagerbehälter überführt, der eine Pufferfunktion aufweist. Zusätzlich kann das Produkt im Lagerbehälter beispielsweise durch ständiges Abführen eines Teilstroms aus dem Lagerbehälter zu einem geeigneten Wärmetauscher, beispielsweise zu einem Plattenwärmetauscher, weiter gekühlt werden, bzw. auf einer geeigneten Lagertemperatur gehalten werden. Es ist durchaus möglich, dass in dem Lagerbehälter eine Nachreaktion stattfinden kann.

Die Rückführung des Produktes in den Lagerbehälter, kann grundsätzlich auf beliebige Weise erfolgen. Es hat sich jedoch in einigen Fällen als vorteilhaft herausgestellt, dass das Produkt derart über ein System aus einer oder mehreren Düsen in den Lagerbehälter rückgeführt wird, dass innerhalb des Lagerbehälters eine entsprechende Durchmischung des gelagerten Produktes stattfindet.

Aus dem Lagerbehälter wird weiterhin kontinuierlich Produkt in einen Stabilisierungsbehälter abgeführt. Dort wird das Produkt mit einer geeigneten Säure, beispielsweise mit H₂SO₄, versetzt. Dabei wird der Katalysator deaktiviert.

Ein im Rahmen der vorliegenden Erfindung einsetzbarer Schlaufenreaktor enthält im Betriebszustand ein Reaktionsgemisch und weist mindestens eine Vorrichtung zum Vermischen des Reaktionsgemischs auf. Die Vorrichtung ist derart angeordnet, dass sie von dem im Schlaufenreaktor befindlichen Reaktionsgemisch während eines Durchlaufs des Schlaufenreaktors mindestens einmal passiert wird. Als Mischvorrichtungen sind sowohl Mischvorrichtungen mit beweglichen Elementen als auch so genannte Statikmischer geeignet, bei denen unbewegliche Strömungswiderstände vorgesehen sind. Als Mischvorrichtungen eignen sich dabei beispielsweise Statikmischer, einfache, mit Öffnungen versehene Platten, Venturivorrichtungen, Düsenmischer, Rührvorrichtungen, sonstige Mischvorrichtungen, Sprühdüsen und dergleichen, sofern sie im Rahmen eines Schlaufenmixers erfolgreich zur Vermischung der Reaktanden eingesetzt werden können. Eine entsprechende Mischvorrichtung kann beispielsweise mit Hilfe externer Energiezufuhr den Mischvorgang durchführen. Es ist jedoch ebenso möglich und im Rahmen der vorliegenden Erfindung in einer weiteren Ausführungsform bevorzugt, wenn die Vermischung der Reaktanden ausschließlich durch die im Schlaufenreaktor im Reaktionsgemisch vorhandene kinetische Energie erfolgt. Sinnvollerweise kann die derart vorliegende kinetische Energie beispielsweise durch Statikmischer verwertet werden. Im Falle des Einsatzes von Statikmischern sind beispielsweise solche geeignet, die einen Betriebsüberdruck von mindestens etwa 10, beispielsweise mindestens etwa 15 oder mindestens etwa 20 bar unter Betriebsbedingungen ohne wesentliche Einschränkungen der Funktionsfähigkeit erlauben. Entsprechende Mischer können aus Kunststoff oder Metall bestehen. Als Kunststoff eignen sich beispielsweise PVC, PP; HDPE, PVDF, PFA oder PTFE. Metallmischer können beispielsweise aus Nickellegierungen, Zirkonium, Titan und dergleichen bestehen. Ebenfalls geeignet sind beispielsweise Rechteckmischer.

Die Umsetzung von Blausäure und Aceton zu Acetoncyanhydrin läuft erfindungsgemäß in Gegenwart eines Katalysators ab. Als Katalysatoren eignen sich grundsätzlich basische Verbindungen, beispielsweise anorganische basische Verbindungen oder organische basische Verbindungen. Geeignet sind beispielsweise beliebige alkalische Verbindungen wie Ammoniak, Natronlauge oder Kalilauge, die die Umsetzung von Aceton und Blausäure zu Acetoncyanhydrin katalysieren können. Zu den geeigneten anorganischen basischen Verbindungen zählen beispielsweise Metalloxide oder Metallhydroxide, insbesondere die Hydroxide der Alkalimetalle oder Alkoholate der Alkalimetalle, wobei unter Alkalimetallen im Rahmen des vorliegenden Textes Lithium, Natrium und Kalium verstanden werden. Eine Durchführung des erfindungsgemäßen Verfahrens mit entsprechenden Verbindungen des Rubidiums und Cäsiums ist sicher möglich, wird jedoch üblicherweise an ökonomischen Erwägungen scheitern.

Im Rahmen einer weiteren und in vielen Fällen bevorzugten Ausführungsform der vorliegenden Erfindung werden zur Durchführung des erfindungsgemäßen Verfahrens organische Basen eingesetzt. Als organische Basen eignen sich grundsätzlich alle organischen Verbindungen, die keinen nachteiligen Einfluss auf die erfindungsgemäße Reaktion ausüben und darüber hinaus, sofern das erfindungsgemäß hergestellte Cyanhydrin im Rahmen eines integrierten, weiterführenden Verfahrens, beispielsweise im Rahmen eines Verfahrens zur Herstellung von Methacrylsäurealkylestern eingesetzt werden soll, entweder leicht aus dem Reaktionsgemisch zu entfernen ist oder, sofern es im Reaktionsgemisch verbleibt, auf die weiterführenden Stufen keinen nachteiligen Einfluss hat.

Besonders bevorzugt sind im Rahmen einer weiteren Ausführungsform der vorliegenden Erfindung Amine als organische Basen. Es hat sich als vorteilhaft herausgestellt, wenn als Katalysator ein organischer Katalysator, insbesondere ein Amin eingesetzt wird. Geeignet sind beispielsweise sekundäre oder tertiäre Amine, wie Diethylamin, Dipropylamin, Triethylamin, Tri-n-propylamin und dergleichen.

Es hat sich gemäß der vorliegenden Erfindung als vorteilhaft herausgestellt, wenn die Zugabe der einzelnen, an der Reaktion beteiligten Stoffe derart erfolgt, dass eine besonders innige Vermischung der Reaktanden mit einem die Reaktion unterstützenden Katalysator so erfolgt, dass, sofern die Notwendigkeit der Zugabe eines Katalysators zu einem bestimmten Zeitpunkt besteht, der Katalysator dem Reaktionsgemisch so zugeführt wird, dass bereits eine möglichst gute Vermischung der miteinander zur Reaktion zu bringenden Verbindungen stattgefunden hat, auf der anderen Seite die Vermischung zwischen Katalysator und den Reaktanden ebenfalls möglichst so schnell und so gründlich stattfindet, dass eine möglichst kurze Zeit bis zur vollständigen bzw. weitgehend vollständigen Homogenisierung verstreicht.

Zu diesem Zweck hat es sich erfindungsgemäß als vorteilhaft herausgestellt, wenn zwischen den Zugabeorten für die Reaktanden, d.h. Aceton und Blausäure, dem Zugabeort des Katalysators und der Mischvorrichtung im Schlaufenreaktor bestimmte Relationen bestehen. So kann es erfindungsgemäß beispielsweise vorteilhaft sein, wenn die Zugabestelle bzw. der Zugabeort der beiden Reaktanden Aceton und Blausäure vergleichsweise nahe beieinander liegen. Beispielsweise kann die Zugabestelle für das Aceton von der Zugabestelle für die Blausäure durch eine Distanz getrennt sein, die weniger als 20 % der Gesamtlänge des Schlaufenreaktors, insbesondere weniger als 10 % der Gesamtlänge des Schlaufenreaktors beträgt.

Diese Anordnung wirkt sich insofern vorteilhaft aus, als nach der Zugabe der Reaktanden möglichst schnell bereits eine Vermischung der Reaktanden stattfinden kann. Dies jedoch ohne dass an dieser Stelle bereits aufgrund einer hohen Katalysatorkonzentration die Reaktion derart schnell abläuft, dass mit der Ausbildung lokaler Überhitzungszonen zu rechnen wäre. Es hat sich erfindungsgemäß weiterhin als vorteilhaft herausgestellt, wenn die Distanz von der Zugabestelle für Aceton oder von der Zugabestelle für Blausäure bis zur Vorrichtung zum Vermischen der Reaktanden im Schlaufenreaktor, wobei es sich vorzugsweise um eine statische Mischvorrichtung handelt, derart gewählt wird, dass sie etwa 50 % bis etwa 75 % der Gesamtlänge des Schlaufenreaktors beträgt.

Es hat sich darüber hinaus im Rahmen der vorliegenden Erfindung als gegebenenfalls vorteilhaft erwiesen, wenn der Zugabeort für den Katalysator derart ausgewählt wird, dass die Distanz zwischen Zugabeort des Katalysators und der Vorrichtung zum Vermischen des Reaktionsgemischs im Schlaufenreaktor höchstens 50 % der Distanz beträgt, die zwischen dem Zugabeort für Aceton oder dem Zugabeort für Blausäure liegt, je nachdem welcher dieser Zugabeorte näher an der Vorrichtung zum Vermischen des Reaktionsgemischs im Schlaufenreaktor liegt. In weiteren bevorzugten Ausführungsformen der vorliegenden Erfindung beträgt die Distanz zwischen Zugabeort und Vorrichtung zum Vermischen des Reaktionsgemischs im Schlaufenreaktor des Katalysators in Relation zum Zugabeort des Acetons oder der Blausäure, je nachdem welcher der beiden Zugabeorte näher an der Vorrichtung zum Vermischen des Reaktionsgemischs im Schlaufenreaktor liegt, höchstens etwa 30 % oder höchstens etwa 20 % oder höchstens etwa 10 % oder höchstens etwa 5 %.

Die oben beschriebene Anordnung der Zugabeorte für die Reaktanden bzw. sonstigen Reaktionshilfstoffe, insbesondere für den Katalysator, weist hinsichtlich der Stabilität der Umsetzung im Schlaufenreaktor eine Reihe von Vorteilen auf. Durch die Wahl des Zugabeorts für Aceton und Blausäure in einem Abstand von der Vorrichtung zur Vermischung des Reaktionsgemischs im Schlaufenreaktor und insbesondere im Abstand vom Zugabeort für den Katalysator, wirkt sich dahingehend vorteilhaft aus, dass vor in Kontakt treten des Katalysators mit dem Reaktionsgemisch bereits eine Vermischung der Reaktanden derart stattfinden kann, dass ein bereits weitgehend homogenes Gemisch auf den Katalysator trifft. Hierdurch werden lokale Konzentrationsmaxima weitgehend vermieden, was für eine gleichmäßigere Reaktion sorgt und damit eine Ausbeutesteigerung aufgrund eines geringeren Verlusts an Acetoncyanhydrin durch Rückspaltung mit sich bringt. Durch die Anordnung des Zugabeorts für den Katalysator in unmittelbarer Nähe der Vorrichtung zum Vermischen des Reaktionsgemischs wird darüber hinaus für eine schnelle Homogenisierung des Reaktionsgemischs und des mit dem zugegebenen Katalysator gesorgt. Auch hierbei hat sich gezeigt, dass durch die deutlich verbesserte Homogenisierung und die schnelle Homogenisierung die Ausbildung von lokalen Konzentrationsgradienten verringert und damit die Ausbeute gesteigert werden kann.

Im Rahmen einer weiteren Ausführungsform der vorliegenden Erfindung hat es sich darüber hinaus als vorteilhaft herausgestellt, wenn der Schlaufenreaktor über mindestens eine innerhalb der Reaktorschlaufe liegende Pumpe verfügt. Im Rahmen einer weiteren Ausführungsform der vorliegenden Erfindung wird die Pumpe derart in den Schlaufenreaktor integriert, dass sie zwischen dem Zugabeort für die Reaktanden Aceton und Blausäure und dem Zugabeort des Katalysators angeordnet wird.

Das Reaktionsgemisch wird im Reaktor derart gekühlt, dass das Reaktionsgemisch während des Betriebs des Schlaufenreaktors eine im Wesentlichen gleich bleibende Temperatur aufweist. Die Temperaturschwankungen betragen dabei vorzugsweise weniger als etwa 20 °C, beispielsweise weniger als etwa 10 oder weniger als etwa 5 °C. Dabei wird vorzugsweise mindestens ein Wärmetauscher derart im Schlaufenreaktor angeordnet, dass das Reaktionsgemisch im Betrieb mindestens einmal pro durchlauf des Schlaufenreaktors den Wärmetauscher durchlaufen kann. gegebenenfalls können auch zwei oder drei Wärmetauscher seriell oder parallel das Reaktionsgemisch kühlen. Die Kühlung erfolgt dabei vorzugsweise mittels Wasser, grundsätzlich ist jedoch auch jedes andere Kühlmittel im Rahmen der vorliegenden Erfindung geeignet. Die Temperatur im Reaktionsgemisch wird auf eine Temperatur zwischen 10 und etwa 60 °C, insbesondere auf eine Temperatur von etwa 30 bis etwa 40 °C eingestellt, wie im vorliegenden Text beschrieben.

Das Reaktionsgemisch kann dem Schlaufenreaktor kontinuierlich oder intermittierend, vorzugsweise jedoch kontinuierlich entzogen werden. Ein dem Schlaufenreaktor entzogenes Reaktionsgemisch weist eine Temperatur auf, die vorteilhafterweise noch weiter erniedrigt werden sollte. Hierzu wird im Rahmen der vorliegenden Erfindung im Rahmen einer weiteren Ausgestaltung des Erfindungsgegenstandes das abgezogene Reaktionsgemisch über einen weiteren Wärmetauscher, vorzugsweise über einen Plattenwärmetauscher auf eine Temperatur von weniger als 10 °C, vorzugsweise auf eine Temperatur von etwa 0 bis etwa 5 °C gekühlt.

Anschließend wird das Gemisch den oben beschriebenen Lagerbehälter überführt, indem beispielsweise eine Nachreaktion stattfinden kann. Der Behälter wird vorzugsweise derart umgewälzt, dass ein Teil des Inhalts dem Behälter ständig entzogen, über einen weiteren Wärmetauscher auf die dem Behälter vorgegebene Temperatur, beispielsweise etwa 0 bis etwa 5 °C gekühlt, und anschließend in den Behälter zurückgegeben wird. Die Rückführung in den Behälter und insbesondere die Einspeisung in das im Behälter bereits vorliegende Gemisch wird im Rahmen einer weiteren Ausführungsform der vorliegenden Erfindung vorzugsweise so gewählt, dass der Strom des zurück geführten Materials für eine Durchmischung des im Behälter vorliegenden Reaktionsprodukts sorgt. Beispielsweise kann eine derartige Rückführung des entnommenen Gemischs in den Behälter über eine entsprechend geeignete Düse erfolgen.

Anschließend wird das im Behälter vorliegende Material, entweder intermittierend oder kontinuierlich, in einen weiteren Behälter überführt, in welchem eine Stabilisierung des Produkts stattfindet. Die Stabilisierung erfolgt im Rahmen einer weiteren Ausführungsform der vorliegenden Erfindung mit einer Säure, insbesondere mit einer Mineralsäure. Geeignet ist beispielsweise Schwefelsäure mit einem Wassergehalt von etwa 0,1 bis etwa 10 %, beispielsweise etwa 0,5 bis etwa 8 oder etwa 1 bis etwa 6 %. Die Zugabemenge für die Säure richtet sich dabei nach einem im Gemisch einzustellenden pH-Wert. Ganz besonders geeignet hat es sich beispielsweise herausgestellt, wenn der pH-Wert auf einen Wert von etwa 1 bis etwa 4, insbesondere etwa 1,5 bis etwa 2,5, beispielsweise 2, eingestellt wird.

Das rohe Acetoncyanhydrin als Reaktionsprodukt wird anschließend einer Reinigung unterzogen. Eine Reinigung über eine Kolonne, insbesondere über eine Rektifikationskolonne mit etwa 10 bis etwa 18 Kolonnenböden hat sich dabei in vielen Fällen als geeignet herausgestellt. Das rohe Acetoncyanhydrin kann der Rektifikationskolonne etwa im oberen Drittel der Kolonne zugefügt werden. Die Temperatur des Acetoncyanhydrins kann dabei grundsätzlich etwa 0 bis etwa 100 °C betragen. Es hat sich in verschiedenen Fällen dabei als günstig erwiesen, wenn eine Vorwärmung des Produkts stattfindet. Vorteilhafterweise erfolgt eine Vorwärmung des Produkts beispielsweise durch Wärmeaustausch mit dem aus der Rektifikationskolonne abgezogenen, gereinigten Acetoncyanhydrin. Bei einem derartigen Wärmeaustausch wird beispielsweise Acetoncyanhydrin mit einer Temperatur von etwa 100 °C aus dem Kolonnensumpf entnommen und im Rahmen eines Wärmeaustauschs mit dem zu reinigenden Acetoncyanhydrin auf eine Temperatur von etwa 60 bis etwa 80° gekühlt, wobei der Wärmeübergang derart eingestellt wird, dass das in die Kolonne einzuführende Acetoncyanhydrin auf etwa die gleiche Temperatur gebracht wird.

Ein weiterer Gegenstand der Erfindung besteht in einem Verfahren zur Herstellung von Acetoncyanhydrin, bei dem ein Reaktionsgemisch enthaltend Aceton und Blausäure in Gegenwart eines Katalysators in einem Schlaufenreaktor unter Bildung von Acetoncyanhydrin als Reaktionsprodukt zur Reaktion gebracht werden und bei dem der Schlaufenreaktor mindestens eine Vorrichtung zum Kühlen des Reaktionsgemischs, mindestens eine Pumpe, mindestens eine Vorrichtung zum Vermischen des Reaktionsgemischs und jeweils mindestens eine Zufuhröffnung zum Zuführen von Blausäure, Aceton und Katalysator aufweist, wobei dem Schlaufenreaktor kontinuierlich oder intermittierend Reaktionsprodukt entzogen wird, das Reaktionsprodukt nach Verlassen des Schlaufenreaktors in einem oder mehreren Wärmetauschern auf eine Temperatur von 0 bis 10 °C gekühlt und mit Schwefelsäure mit einen Wassergehalt von 0,1 bis 8 Gew.-% versetzt wird.

Es hat sich erfindungsgemäß gezeigt, das sich die Ausbeute an Acetoncyanhydrin durch Behandlung eines Reaktionsprodukts, wie es gemäß der vorliegenden Erfindung erhalten wird, mit Schwefelsäure mit einen Wassergehalt von 0,1 bis 8 Gew.-%, beispielsweise mit einem Wassergehalt von etwa 0,5 bis etwa 7 oder etwa 1 bis etwa 6 oder etwa 2 bis etwa 5 oder etwa 3 bis etwa 4,5, beispielsweise 4 Gew.-% eine ausgezeichnete Stabilisierung erzielen lässt. Darüber hinaus eignet sich eine derartige Behandlung als Vorbereitung für den Einsatz in weiterführenden Verfahren, insbesondere als Vorbereitung zum Einsatz in einem integrierten Verfahren zur Herstellung von Methacrylsäurealkylestern, bei dem als ein nächster Schritt beispielsweise die Amidierung des Acetoncyanhydrins erfolgt.

Die im Rahmen der vorliegenden Erfindung vorgeschlagenen Maßnahmen eignen sich sowohl einzeln als auch getrennt zur Verbesserung der Ausbeute in Verfahren zur Herstellung von Acetoncyanhydrin. Die Maßnahmen können dabei in beliebiger Kombination angewandt werden, die Lehre des vorliegenden Textes umfasst dabei ausdrücklich auch derartige Kombinationen.

Das erfindungsgemäße Verfahren weist eine Reihe von Vorteilen insbesondere dahingehend auf, als es sich besonders gut zur Einbindung in ein Verfahren zur Herstellung Methacrylsäurealkylestern eignet. Das vorliegende Verfahren führt zu besonders reinen Produkten in hoher Ausbeute. Gegenstand der vorliegenden Erfindung ist daher auch ein Verfahren zur Herstellung von Methacrylsäurealkylester, das in mindestens einem Schritt Acetoncyanhydrin einsetzt, wie es gemäß der vorliegenden Erfindung erhalten werden kann.

Nachfolgend werden verschiedene Verfahrenselemente erläutert, die grundsätzlich einzeln oder als Ensemble aus zwei oder mehr der genannten Verfahrenselemente mit der vorliegenden Erfindung verbindbar sind. Gegebenenfalls kann es vorteilhaft sein, wenn die im Rahmen des vorliegenden Textes vorgestellten Verfahrenselemente derart mit der vorliegenden Erfindung kombiniert werden, dass sie insgesamt zu einem Verfahren zur Herstellung von Estern der Methacrylsäure oder einem Verfahren zur Herstellung von Methacrylsäure kombiniert werden. Es sei jedoch auch darauf hingewiesen, dass sich zumeist vorteilhafte Effekte auch dann erzielen lassen, wenn der Gegenstand der vorliegenden Erfindung als solcher in einem anderen Umfeld eingesetzt wird oder nur mit einem Teil der hier vorgestellten Verfahrenselemente kombiniert wird.

### ACH-Aufarbeitung

Im Rahmen eines weiteren Verfahrenselementes, das im Zusammenhang mit der vorliegenden Erfindung eingesetzt werden kann, wird Acetoncyanhydrin, das in einer vorgelagerten Stufe beispielsweise aus der Umsetzung von Aceton mit Blausäure erhalten wurden, einer destillativen Aufarbeitung unterzogen. Dabei wird das stabilisierte rohe Acetoncyanhydrin über eine entsprechende Kolonne von niedrig siedenden Bestandteilen befreit. Ein geeignetes Destillationsverfahren kann beispielsweise über nur eine Kolonne geführt werden. Es ist jedoch ebenfalls möglich, im Rahmen einer entsprechenden Aufreinigung von rohem Acetoncyanhydrin eine Kombination von zwei oder mehr Destillationskolonnen auch kombiniert mit einem Fallfilmverdampfer einzusetzen. Weiterhin könne zwei oder mehrere Fallfilmverdampfer oder auch zwei oder mehrere Destillationskolonnen miteinander kombiniert werden.

Das rohe Acetoncyanhydrin kommt in der Regel mit einer Temperatur von etwa 0 bis etwa 15 °C, beispielsweise einer Temperatur von etwa 5 bis etwa 10 °C aus der Lagerung zur Destillation. Grundsätzlich kann das rohe Acetoncyanhydrin direkt in die Kolonne eingeführt werden. Es hat sich jedoch in einigen Fällen bewährt, wenn zunächst das rohe, kühle Acetoncyanhydrin über einen Wärmetauscher einen Teil der Wärme des bereits destillativ gereinigten Produktes übernimmt. Daher wird im Rahmen einer weiteren Ausführungsform des hier beschriebenen Verfahrens das rohe Acetoncyanhydrin über einen Wärmetauscher auf eine Temperatur von etwa 60 bis 80 °C erhitzt.

Die destillative Reinigung des Acetoncyanhydrin erfolgt über eine Destillationskolonne mit mehr als 10 Böden oder über eine Kaskade von zwei oder mehr entsprechend geeigneter Destillationskolonnen. Die Beheizung des Kolonnensumpfes erfolgt vorzugsweise mit Dampf. Es hat sich als vorteilhaft herausgestellt, wenn die Sumpftemperatur eine Temperatur von 140 °C nicht übersteigt, gute Ausbeuten und eine gute Reinigung haben sich erzielen lassen, wenn die Sumpftemperatur nicht größer als etwa 130 °C oder nicht höher als etwa 110 °C ist. Die Temperaturangaben beziehen sich dabei auf die Wandtemperatur des Kolonnensumpfes.

Das rohe Acetoncyanhydrin wird im oberen Drittel der Kolonne dem Kolonnenkörper zugeführt. Die Destillation wird vorzugsweise bei verringertem Druck, beispielsweise bei einem Druck von etwa 50 bis etwa 900 mbar, insbesondere etwa 50 bis etwa 250 mbar und mit guten Ergebnissen zwischen 50 bis etwa 150 mbar durchgeführt.

Am Kopf der Kolonne werden gasförmige Verunreinigungen, insbesondere Aceton und Blausäure, entnommen, die abgetrennten gasförmigen Stoffe werden über einen Wärmetauscher oder eine Kaskade von zwei oder mehr Wärmetauschern gekühlt. Hierbei wird vorzugsweise eine Solekühlung mit einer Temperatur von etwa 0 bis etwa 10 °C eingesetzt. Dabei wird den gasförmigen Inhaltsstoffen der Brüden die Gelegenheit gegeben, zu kondensieren. Die erste Kondensationsstufe kann beispielsweise bei Normaldruck stattfinden. Es ist jedoch ebenso möglich und hat sich in einigen Fällen als vorteilhaft erwiesen, wenn diese erste Kondensationsstufe unter verringertem Druck, vorzugsweise bei dem Druck, der im Rahmen der Destillation vorherrscht, erfolgt. Das Kondensat wird in einen gekühlten Auffangbehälter weitergeleitet und dort bei einer Temperatur von etwa 0 bis etwa 15 °C, insbesondere bei etwa 5 bis etwa 10 °C gesammelt.

Die im Rahmen des ersten Kondensationsschrittes nicht kondensierenden gasförmigen Verbindungen werden über eine Vakuumpumpe aus dem Unterdruckraum entfernt. Hierbei ist grundsätzlich eine beliebige Vakuumpumpe einsetzbar. Es hat sich jedoch in vielen Fällen als vorteilhaft erwiesen, wenn eine Vakuumpumpe eingesetzt wird, die aufgrund ihrer Bauweise nicht zum Eintrag flüssiger Verunreinigungen in den Gasstrom führt. Vorzugsweise werden hier daher beispielsweise trocken laufende Vakuumpumpen eingesetzt.

Der auf der Druckseite der Pumpe entweichende Gasstrom wird über einen weiteren Wärmetauscher geführt, der vorzugsweise mit Sole bei einer Temperatur von etwa 0 bis etwa 15 °C gekühlt wird. Hierbei kondensierende Inhaltsstoffe werden ebenfalls in dem Sammelbehälter gesammelt, der bereits die unter Vakuumbedingungen gewonnenen Kondensate auffängt. Die auf der Druckseite der Vakuumpumpe durchgeführte Kondensation kann beispielsweise durch einen Wärmetauscher, jedoch auch mit einer Kaskade von zwei oder mehr seriell parallel angeordneten Wärmetauschern erfolgen. Nach diesem Kondensationsschritt verbleibende gasförmigen Stoffen werden abgeführt und einer beliebigen weiteren Verwertung, beispielsweise einer thermischen Verwertung, zugeführt.

Die gesammelten Kondensate können ebenfalls beliebig weiterverwertet werden. Es hat sich jedoch als unter ökonomischen Gesichtspunkten äußerst Vorteilhaft erwiesen, die Kondensate in die Reaktion zur Herstellung von Acetoncyanhydrin zurückzuführen. Dies erfolgt vorzugsweise an einer oder mehreren Stellen, die Zugang zum Schlaufenreaktor ermöglichen. Die Kondensate können grundsätzlich eine beliebige Zusammensetzung aufweisen, sofern sie die Herstellung des Acetoncyanhydrins nicht stören. In vielen Fällen wird die überwiegende Menge des Kondensates jedoch aus Aceton und Blausäure bestehen, beispielsweise in einem molaren Verhältnis von etwa 2:1 bis etwa 1:2, häufig in einem Verhältnis von etwa 1:1.

Das aus dem Sumpf der Destillationskolonne gewonnene Acetoncyanhydrin wird zunächst über einen ersten Wärmetauscher durch das zugeführte, kalte rohe Acetoncyanhydrin auf eine Temperatur von etwa 40 bis etwa 80 °C abgekühlt. Anschließend wird das Acetoncyanhydrin über einen mindestens einen weiteren Wärmetauscher auf eine Temperatur von etwa 30 bis etwa 35 °C gekühlt und ggf. zwischengelagert.

### Amidierung

Im Rahmen eines weiteren Verfahrenselements, wie er häufig bei der Herstellung von Methacrylsäure oder von Estern der Methacrylsäure vorgesehen ist, wird Acetoncyanhydrin einer Hydrolyse unterzogen. Dabei bildet sich bei verschiedenen Temperaturstufen nach einer Reihe von Reaktionen als Produkt Methacrylamid.

Die Umsetzung wird in einer dem Fachmann bekannten Weise durch eine Reaktion zwischen konzentrierter Schwefelsäure und Acetoncyanhydrin bewirkt. Die Umsetzung ist exotherm, so dass in vorteilhafter Weise Reaktionswärme aus dem System abgeführt wird.

Die Umsetzung kann auch hier wieder im Batchverfahren oder in kontinuierlichen Verfahren durchgeführt werden. Letzteres hat sich in vielen Fällen als vorteilhaft erwiesen. Sofern die Umsetzung im Rahmen eines kontinuierlichen Verfahrens durchgeführt wird, hat sich der Einsatz von Schlaufenreaktoren bewährt. Die Umsetzung kann beispielsweise in nur einem Schlaufenreaktor erfolgen. Es kann jedoch vorteilhaft sein, wenn die Umsetzung in einer Kaskade von zwei oder mehreren Schlaufenreaktoren durchgeführt wird.

Ein geeigneter Schlaufenreaktor weist im Rahmen des beschriebenen Verfahrens eine oder mehrere Zufuhrstellen für Acetoncyanhydrin, eine oder mehrere Zufuhrstellen für konzentrierte Schwefelsäure, einen oder mehrere Gasabscheider, einen oder mehrere Wärmetauscher und eine oder mehrere Mischer auf.

Die Hydrolyse von Acetoncyanhydrin mit Schwefelsäure zu Methacrylamid ist, wie bereits beschrieben, exotherm. Die im Rahmen der Reaktion anfallende Reaktionswärme muss dem System jedoch zumindest weitgehend entzogen werden, da mit zunehmender Temperatur bei der Umsetzung die Ausbeute sinkt. Zwar ist es grundsätzlich möglich mit entsprechenden Wärmetauschern eine schnelle und umfassende Abfuhr der Reaktionswärme zu erzielen. Es kann jedoch auch nachteilig sein, das Gemisch zu sehr zu kühlen, da für einen entsprechenden Austausch an den Wärmetauschern ein ausreichender Wärmeübergang erforderlich ist. Da mit sinkender Temperatur die Viskosität des Gemischs stark ansteigt, wird so einerseits die Zirkulation im Schlaufenreaktor erschwert, andererseits lässt sich eine ausreichende Abfuhr der Reaktionsenergie aus dem System nicht mehr gewährleisten.

Darüber hinaus können zu geringe Temperaturen im Reaktionsgemisch zu einer Kristallisation von Inhaltsstoffen des Reaktionsgemischs an den Wärmetauschern führen. Dadurch wird der Wärmeübergang weiter verschlechtert, wodurch sich eindeutiger Ausbeuterückgang verzeichnen lässt. Darüber hinaus kann der Schlaufenreaktor nicht mit den optimalen Mengen an Reaktanden beschickt werden, so dass insgesamt die Effizienz des Verfahrens leidet.

Im Rahmen einer Ausgestaltung des Verfahrens wird aus einem Strom von Acetoncyanhydrin ein Teil, vorzugsweise etwa zwei Drittel bis etwa drei Viertel, des Volumenstroms in einen ersten Schlaufenreaktor eingeführt. Vorzugsweise weist ein erster Schlaufenreaktor einen oder mehrere Wärmetauscher, eine oder mehrere Pumpen, einen oder mehrere Mischelemente und einen oder mehrere Gasabscheider auf. Die den ersten Schlaufenreaktor durchlaufenden Umwälzströme liegen vorzugsweise im Bereich von etwa 100 bis 450 m³/h, bevorzugt in einem Bereich von 200 bis 400 m³/h und darüber hinaus bevorzugt in einem Bereich von etwa 250 bis 350 m³/h. In einen auf den ersten Schlaufenreaktor folgenden mindestens einen weiteren Schlaufenreaktor liegen die Umwälzströme vorzugsweise in einem Bereich von etwa 40 bis 450 m³/h, bevorzugt in einem Bereich von 50 bis 400 m³/h und darüber hinaus bevorzugt in einem Bereich von etwa 60 bis 350 m³/h. Weiterhin sind als Temperaturdifferenz über den Wärmetauscher etwa 1 bis 10° C bevorzugt, wobei etwa 2 bis 7 °C besonders bevorzugt sind.

Die Zufuhr des Acetoncyanhydrin kann grundsätzlich an beliebiger Stelle in den Schlaufenreaktor erfolgen. Es hat sich jedoch als vorteilhaft erwiesen, wenn die Zufuhr in ein Mischelement, beispielsweise in einen Mischer mit beweglichen Teilen oder einen Statikmischer erfolgt. Die Zufuhr der Schwefelsäure erfolgt vorteilhafter Weise vor der Acetoncyanhydrinzugabe. Ansonsten ist es jedoch ebenfalls möglich, die Schwefelsäure an beliebiger Stelle in den Schlaufenreaktor einzuführen.

Das Verhältnis der Reaktanden im Schlaufenreaktor wird so gesteuert, dass ein Überschuss Schwefelsäure vorliegt. Der Überschuss an Schwefelsäure beträgt, bezüglich des molaren Verhältnisses der Inhaltsstoffe, im ersten Schlaufenreaktor etwa 1,8:1 bis etwa 3:1 und im letzten Schlaufenreaktor etwa 1,3:1 bis etwa 2:1.

In einigen Fällen hat es sich als vorteilhaft erwiesen, mit einem derartigen Überschuss an Schwefelsäure die Reaktion im Schlaufenreaktor zu betreiben. Die Schwefelsäure kann hier beispielsweise als Lösemittel dienen und die Viskosität des Reaktionsgemischs niedrig halten, wodurch eine höhere Abfuhr von Reaktionswärme und eine niedrigere Temperatur des Reaktionsgemischs gewährleistet werden kann. Dies kann deutliche Ausbeutevorteile mit sich bringen. Die Temperatur im Reaktionsgemisch beträgt etwa 90 bis etwa 120 °C.

Die Wärmeabfuhr wird durch einen oder mehrere Wärmetauscher im Schlaufenreaktor gewährleistet. Es hat sich dabei als vorteilhaft erwiesen, wenn die Wärmetauscher über eine geeignete Sensorik zum Einstellen der Kühlleistung verfügen, um eine zu starke Kühlung des Reaktionsgemischs aus den oben genannten Gründen zu verhindern. So kann es beispielsweise vorteilhaft sein, den Wärmeübergang im Wärmetauscher oder in den Wärmetauschern punktförmig oder kontinuierlich zu messen und daran die Kühlleistung der Wärmetauscher anzupassen. Dies kann beispielsweise über das Kühlmittel selbst geschehen. Es ist auch ebenso möglich durch entsprechende Variation der Zugabe der Reaktionspartner und durch die Erzeugung von mehr Reaktionswärme eine entsprechenden Erhitzung des Reaktionsgemischs zu erreichen. Auch eine Kombination von beiden Möglichkeiten ist denkbar. Der Schlaufenreaktor sollte darüber hinaus über mindestens einen Gasabscheider verfügen. Über den Gasabscheider wird einerseits dem Schlaufenreaktor kontinuierlich gebildetes Produkt entnommen. Andererseits lassen sich im Rahmen der Reaktion gebildete Gase so dem Reaktionsraum entziehen. Als Gas bildet sich hauptsächlich Kohlenmonoxid. Das aus dem Schlaufenreaktor entnommene Produkt wird vorzugsweise in einen zweiten Schlaufenreaktor überführt. In diesen zweiten Schlaufenreaktor wird das Schwefelsäure und Methacrylsäureamid beinhaltende Reaktionsgemisch wie es durch die Reaktion im ersten Schlaufenreaktor erhalten wurde, mit dem verbleibenden Teilstrom an Acetoncyanhydrin umgesetzt. Hierbei reagiert der Überschuss an Schwefelsäure aus dem ersten Schlaufenreaktor, oder zumindest ein Teil der überschüssigen Schwefelsäure, mit dem Acetoncyanhydrin unter weiterer Bildung von Methacrylsäureamid. Die Durchführung der Reaktion in zwei oder mehr Schlaufenreaktoren weist den Vorteil auf, dass aufgrund des Schwefelsäureüberschusses im ersten Schlaufenreaktor die Pumpbarkeit des Reaktionsgemischs und damit der Wärmeübergang und letztendlich die Ausbeute verbessert werden. Im zweiten Schlaufenreaktor ist wiederum mindestens ein Mischelement, mindestens ein Wärmetauscher und mindestens ein Gasabscheider angeordnet. Die Reaktionstemperatur im zweiten Schlaufenreaktor beträgt ebenfalls etwa 90 bis etwa 120 °C.

Das Problem der Pumpbarkeit des Reaktionsgemischs, des Wärmeübergangs und einer möglichst geringen Reaktionstemperatur stellt sich im jedem weiteren Schlaufenreaktor genauso wie im ersten. Deshalb verfügt vorteilhafter Weise auch der zweite Schlaufenreaktor über einen Wärmetauscher, dessen Kühlleistung durch entsprechende Sensorik geregelt werden kann.

Die Zufuhr des Acetoncyanhydrins erfolgt wiederum in einem geeigneten Mischelement, vorzugsweise in einen Statikmischer.

Aus dem Gasabscheider des zweiten Schlaufenreaktors wird das Produkt entnommen und zur Vervollständigung der Umsetzung und zur Bildung des Methacrylsäureamids auf eine Temperatur von etwa 140 bis etwa 180 °C erhitzt.

Die Erhitzung wird vorzugsweise derart durchgeführt, dass die Maximaltemperatur nur für einen möglichst kurzen Zeitraum, beispielsweise für eine Zeit von etwa einer Minute bis etwa 30 Minuten, insbesondere für eine Zeit von etwa zwei bis etwa acht oder etwa drei bis etwa fünf Minuten erreicht wird. Dies kann grundsätzlich in beliebigen Apparaturen zur Erzielung einer derartigen Temperatur für einen derart kurzen Zeitraum erfolgen. Beispielsweise kann die Energiezufuhr auf konventionellem Wege durch elektrische Energie oder durch Dampf erfolgen. Es ist jedoch ebenso möglich, die Energie durch elektromagnetische Strahlung, beispielsweise durch Mikrowellen zuzuführen.

Es hat sich in verschiedenen Fällen als vorteilhaft erwiesen, wenn der Erhitzungsschritt in einem Wärmetauscher mit zwei oder mehrstufiger Anordnung von Rohrwendeln, die vorzugsweise in einer mindestens doppelten, gegenläufigen Anordnung vorliegen können, erfolgt. Dabei wird das Reaktionsgemisch schnell auf eine Temperatur von etwa 140 bis 180 °C erhitzt.

Der Wärmetauscher kann beispielsweise mit einem oder mehreren Gasabscheidern kombiniert werden. So ist es beispielsweise möglich, das Reaktionsgemisch nach Verlassen der ersten Rohrwendel im Wärmetauscher durch einen Gasabscheider zu führen. Dabei können beispielsweise während der Reaktion entstehende, gasförmige Komponenten aus dem Reaktionsgemisch abgetrennt werden. Es ist ebenso möglich, das Reaktionsgemisch nach Verlassen der zweiten Wendel mit einem Gasabscheider zu behandeln. Es kann sich darüber hinaus als vorteilhaft erweisen, an beiden Stellen, sowohl nach Verlassen der ersten als auch nach Verlassen der zweiten Rohrwendel das Reaktionsgemisch mit einem Gasabscheider zu behandeln.

Die so erhältliche Amidlösung weist in der Regel eine Temperatur von mehr als 100 °C, üblicherweise eine Temperatur von etwa 140 bis 180 °C auf.

Die im Rahmen der Amidierung anfallenden gasförmigen Verbindungen können grundsätzlich in beliebiger Weise entsorgt oder einer Weiterverarbeitung zugeführt werden. Es kann in einigen Fällen jedoch vorteilhaft sein, wenn die entsprechenden Gase in einer Transportleitung dergestalt zusammengeführt werden, dass sie entweder kontinuierlich oder bei Bedarf ggf. mit Druck, beispielsweise mit Dampfdruck, beaufschlagt und so weitertransportiert werden können.

### Veresterung

Ein weiterer ein Verfahrenselement darstellender Schritt, der im Zusammenhang mit dem erfindungsgemäßen Verfahren eingesetzt werden kann, ist eine Hydrolyse von Methacrylsäureamid zu Methacrylsäure und deren gleichzeitiger Veresterung zu Methacrylsäureester. Diese Reaktion kann in einem oder mehreren beheizten, beispielsweise durch Dampf beheizten Kesseln, durchgeführt werden. Es hat sich in vielen Fällen als vorteilhaft erwiesen, wenn die Veresterung in mindestens zwei aufeinanderfolgenden Kesseln, beispielsweise jedoch auch in drei oder vier oder mehr aufeinander folgenden Kesseln durchgeführt wird. Dabei wird eine Lösung von Methacrylsäureamid in den Kessel bzw. in den ersten Kessel einer zwei oder mehr Kessel umfassenden Kaskade von Kesseln eingeführt.

Es ist häufig bevorzugt, eine entsprechende Veresterungsreaktion mit einer Kaskade von zwei oder mehr Kesseln durchzuführen. Im Folgenden soll daher ausschließlich auf diese Variante Bezug genommen werden.

Im Rahmen des hier beschriebenen Verfahrens kann beispielsweise eine Amidlösung, wie sie aus der hier beschriebenen Amidierungsreaktion erhältlich ist, in einen ersten Kessel eingespeist werden. Der Kessel wird beispielsweise mit Dampf beheizt. Die zugeführte Amidlösung weist in der Regel eine erhöhte Temperatur auf, beispielsweise eine Temperatur von etwa 100 bis etwa 180 °C, im Wesentlichen entsprechend der Ablauftemperatur der Amidlösung aus der oben vorgestellten Amidierungsreaktion. Den Kesseln wird weiterhin ein Alkanol zugeführt, das zur Veresterung eingesetzt werden kann.

Grundsätzlich eignen sich hier beliebige Alkanole mit 1 bis etwa 4 Kohlenstoffatomen, die linear oder verzweigt, gesättigt oder ungesättigt sein können, wobei Methanol besonders bevorzugt ist. Ebenso können diese Alkanole zusammen mit Methacrylsäureestern eingesetzt werden, was insbesondere bei Umesterungen der Fall ist.

Der Kessel wird weiterhin mit Wasser beschickt, so dass insgesamt eine Wasserkonzentration im Kessel von etwa 13 bis etwa 26 Gew.-%, insbesondere etwa 18 bis etwa 20 Gew.-% herrscht.

Die Menge an Amidlösung und an Alkanol wird derart geregelt, dass ein Gesamtmolverhältnis von Amid zu Alkanol von etwa 1:1,4 bis etwa 1:1,6 herrscht. Das Alkanol kann auf die Kesselkaskade derart verteilt werden, dass im ersten Reaktor das Molverhältnis etwa 1:1,1 bis etwa 1:1,4 beträgt und in den folgenden Reaktionsstufen bezogen auf den Gesamtamidstrom Molverhältnisse von etwa 1:0,05 bis etwa 1:0,3 eingestellt werden. Das in die Veresterung zugeführte Alkanol kann sich aus "Frischalkanol" sowie Alkanol aus Recyclingströmen der Aufarbeitungsstufen und bei Bedarf auch aus Recyclingströmen der Downstreamprozesse des Produktionsverbundes zusammen setzen.

Die Beschickung des ersten Kessels mit Wasser kann grundsätzlich dahingehend erfolgen, dass Wasser aus einer beliebigen Quelle dem Kessel zugeführt wird, sofern dieses Wasser keine Inhaltsstoffe aufweist, die die Veresterungsreaktion oder die nachfolgenden Prozessstufen nachteilig beeinflussen könnten. Beispielsweise kann dem Kessel VE-Wasser oder Brunnenwasser zugeführt werden. Es ist jedoch ebenfalls möglich, dem Kessel ein Gemisch aus Wasser und organischen Verbindungen zuzuführen, wie sie beispielsweise bei der Reinigung von Methacrylsäure oder Methacrylsäureestern anfallen. Im Rahmen einer bevorzugten Ausführungsform des hier vorgestellten Verfahrens werden die Kessel zumindest anteilig mit einem Gemisch aus Wasser und solchen organischen Verbindungen beschickt.

Wenn eine Kaskade von zwei oder mehr Kesseln im Rahmen der Veresterungsreaktion eingesetzt wird, so können die entstandenen gasförmigen Stoffe, insbesondere der Methacrylsäureester, grundsätzlich aus jedem Kessel einzeln abgezogen und einer Reinigung zugeführt werden. Es hat sich jedoch in manchen Fällen als vorteilhaft erwiesen, wenn bei einer Kaskade von zwei oder mehr Kesseln die gasförmigen Produkte aus dem ersten Kessel zunächst in den zweiten Reaktionskessel eingespeist werden, ohne dass die gasförmigen Verbindungen aus dem ersten Kessel direkt einer Reinigung zugeführt werden. Diese Vorgehensweise bietet den Vorteil, dass der im ersten Kessel häufig starken Schaumentwicklung nicht durch aufwendige, apparative Entschäumung begegnet werden muss. Im Falle einer Kaskadierung der gasförmigen Stoffe aus dem ersten Kessel in den zweiten Kessel tritt der im ersten Kessel gebildete und ggf. mitgerissene Schaum einfach in den Reaktionsraum des zweiten Kessels mit ein. Da dort in der Regel die Schaumbildung deutlich geringer ist, muss so nicht apparativ entschäumt werden.

Der nach einem ersten Kessel angeordnete zweite Kessel nimmt nun einerseits den Überlauf des ersten Kessels auf, andererseits wird er mit den gasförmigen im ersten Kessel gebildeten oder im ersten Kessel vorhandenen Stoffen gespeist. Der zweite Kessel und die eventuell. folgenden werden ebenfalls mit Methanol beschickt. Hierbei ist es bevorzugt, dass die Methanolmenge von Kessel zu Kessel um mindestens 10 %, jeweils auf den vorhergehenden Kessel bezogen, abnimmt. Die Wasserkonzentration im zweiten Kessel sowie in den weiteren Kesseln kann sich von der des ersten Kessels unterscheiden, oftmals sind die Konzentrationsunterschied jedoch gering.

Die im zweiten Kessel entstehenden Brüden werden aus dem Kessel abgeführt und in den Sumpf einer Destillationskolonne eingeleitet.

Wenn die Veresterung mit einer Kaskade von drei oder mehr Kesseln durchgeführt wird, so wird jeweils der Überlauf des zweiten Kessels in einen dritten Kessel überführt sowie der Überlauf des dritten Kessels ggf. in einen vierten Kessel überführt. Die weiteren Kessel werden ebenfalls dampfbeheizt. Vorzugsweise wird die Temperatur in den Kesseln 3 und ggf. 4 auf etwa 120 °C bis etwa 140 °C eingestellt.

Die aus den Kesseln entweichenden Brüden werden in eine Destillationskolonne eingeleitet, wobei diese vorzugsweise im Unterbereich der Destillationskolonne erfolgt. Die Brüden umfassen ein azeotropes Gemisch aus Träger-Dampf, Methacrylsäureester und Alkanol und weisen je nach eingesetztem Alkanol eine Temperatur von etwa 60 bis etwa 120 °C, beispielsweise etwa 70 bis etwa 90 °C bei Einsatz von Methanol auf. In der Destillationskolonne wird der Methacrylsäureester gasförmig von den bei höheren Temperaturen siedenden Brüdenbestandteilen abgetrennt. Die hochsiedenden Anteile (hauptsächlich Methacrylsäureamid, Hydroxyisobuttersäureester und Wasser) werden in den ersten Reaktionskessel zurückgeführt. Der gebildete Methacrylsäureester wird am Kolonnenkopf abgezogen und über einen Wärmetauscher oder eine Kaskade von zwei oder mehr Wärmetauschern abgekühlt. Es hat sich in einigen Fällen bewährt, wenn die Kühlung des Methacrylsäureesters über mindestens zwei Wärmetauscher erfolgt, wobei ein erster Wärmetauscher mit Wasser die Kondensation und eine Kühlung auf eine Temperatur von etwa 60 bis etwa 30 °C durchführt, während ein zweiter, solegekühlter Wärmetauscher eine Abkühlung auf etwa 5 bis etwa 15 °C vornimmt. Von dem wassergekühlten Kondensat kann ein Teilstrom als Rücklauf auf die Kolonnen zur Konzentrationssteuerung der Kolonne gegeben. Es ist jedoch ebenso möglich, den gebildeten Methacrylsäureester über eine Kaskade von mehr als zwei Wärmetauschern zu kühlen. Hierbei ist es beispielsweise möglich, zunächst eine Kühlung über zwei hintereinander geschaltete, wassergekühlte Wärmetauscher vorzunehmen und anschließend über einen entsprechenden solegekühlten Wärmetauscher eine weitere Abkühlung zu erzielen.

So kann beispielsweise im Rahmen des hier vorgestellten Verfahrens der gebildete Methacrylsäureester in gasförmigem Zustand über einen ersten Wärmetauscher mit Wasserkühlung gekühlt werden. Sowohl kondensierte als auch nicht kondensierte Stoffe werden anschließend in einen zweiten Wärmetauscher weitergeleitet, wo eine weitere Kondensation über Wasserkühlung stattfindet. An dieser Stelle können nun beispielsweise gasförmige Stoffe in einen separaten, mit Sole gekühlten Wärmetauscher überführt werden. Das Kondensat in diesem solegekühlten Wärmetauscher wird anschließend in den Destillatstrom gegeben, während die verbleibenden gasförmigen Stoffe weiter verwertet werden können oder einer Entsorgung zugeführt werden. Das Methacrylsäureester-Kondensat aus dem zweiten wassergekühlten Wärmetauscher wird nun in einem mit Wasser oder mit Sohle gekühlten Wärmetauscher auf eine Temperatur von weniger als 15 °C, vorzugsweise etwa 8 bis etwa 12 °C gekühlt. Dieser Kühlschritt kann dazu führen, dass der gebildete Methacrylsäureester einen deutlich niedrigeren Gehalt an Ameisensäure aufweist, als dies ohne den entsprechenden Kühlungsschritt der Fall wäre. Das abgekühlte Kondensat wird anschließend in einen Phasentrenner überführt. Hier wird die organische Phase (Methacrylsäureester) von der wässrigen Phase getrennt. Die wässrige Phase, die neben Wasser noch einen Gehalt an organischen Verbindungen, insbesondere Alkanol, aus dem Destillationsschritt aufweisen kann, kann grundsätzlich beliebig weiterverwendet werden. Wie jedoch bereits oben beschrieben, kann es bevorzugt sein, dieses Gemisch aus Wasser und organischen Verbindungen wieder in den Veresterungsprozess zurückzuführen, indem eine Einspeisung in den ersten Reaktionskessel erfolgt.

Die abgetrennte organische Phase wird in einen Wäscher eingespeist. Dort wird der Methacrylsäureester mit demineralisiertem Wasser gewaschen. Die abgeschiedene wässrige Phase, die ein Gemisch aus Wasser und organischen Verbindungen, insbesondere Alkanol, enthält, kann wiederum grundsätzlich beliebig weiterverwendet werden. Es ist jedoch unter ökonomischen Gesichtspunkten vorteilhaft, diese wässrige Phase wieder in den Veresterungsschritt rückzuführen, indem sie beispielsweise in den ersten Kessel eingespeist wird.

Da Methacrylsäureester eine starke Neigung zur Polymerisation aufweisen, ist es in vielen Fällen vorteilhaft, wenn im Rahmen der Veresterung von Methacrylsäure dafür Sorge getragen wird, dass eine derartige Polymerisation verhindert wird.

In Anlagen zur Herstellung von Methacrylsäure oder Methacrylsäureestern findet Polymerisation oft dann statt, wenn Methacrylsäure oder Methacrylsäureester einerseits eine geringe Strömungsgeschwindigkeit aufweisen, so dass sich lokal Ruhezonen ausbilden können, in denen ein über längere Zeit andauernder Kontakt zwischen Methacrylsäure oder Methacrylsäureester und einem Polymerisationsinitiator einstellen kann, der in Folge dann zur Polymerisation führen kann.

Um ein entsprechendes Polymerisationsverhalten zu vermeiden, kann es vorteilhaft sein, eine Optimierung des Stoffflusses dahingehend durchzuführen, dass einerseits die Strömungsgeschwindigkeit des Methacrylsäureesters oder der Methacrylsäure an möglichst allen Stellen im System so hoch ist, dass die Zahl der Ruhezonen minimiert wird. Darüber hinaus kann es vorteilhaft sein, den Strom an Methacrylsäure bzw. Methacrylsäureester derart mit geeigneten Stabilisatoren zu versetzen, dass eine Polymerisation weitgehend unterdrückt wird.

Zu diesem Zweck können im Rahmen des hier dargestellten Verfahrens grundsätzlich die Stoffströme derart mit Stabilisatoren versetzt werden, dass möglichst wenig Polymerisation im System selbst stattfindet. Hierzu wird insbesondere der Teil der Anlage mit entsprechenden Stabilisatoren versorgt, in dem die Methacrylsäure bzw. der Methacrylsäureester während oder nach der Destillation in hoher Konzentration vorliegt.

So hat es sich beispielsweise als sinnvoll erwiesen, am Kopf der Destillationskolonne dem dort abgezogenen Strom an Methacrylsäureester einen Stabilisator zuzuführen. Weiterhin hat es sich als vorteilhaft erwiesen, diejenigen Anlagenteile mit einer Lösung von Stabilisator in Methacrylsäureester zu spülen, in denen Methacrylsäure oder Methacrylsäureester mit einer Temperatur von mehr als etwa 20 °C, vorzugsweise mit einer Temperatur im Bereich von etwa 20 bis etwa 120 °C zirkuliert. So wird beispielsweise ein Teil des in den Wärmetauschern anfallenden Kondensates zusammen mit einem geeigneten Stabilisator derart in den Kopf der Destillationskolonne zurückgeführt, dass dort der Kolonnenkopf an seiner Innenseite ständig mit stabilisiertem Methacrylsäureester oder stabilisierter Methacrylsäure besprüht wird. Dies geschieht vorzugsweise derart, dass sich im Kolonnenkopf keine Ruhezonen ausbilden können, an denen eine Polymerisation von Methacrylsäure oder Methacrylsäureester zu befürchten ist. Die Wärmetauscher selbst können entsprechend ebenfalls mit einer stabilisierten Lösung von Methacrylsäure oder Methacrylsäureester derart beaufschlagt werden, dass auch hier keine Ruhezonen zur Ausbildung gelangen können.

Es hat sich weiterhin als Vorteil im Rahmen des hier vorgestellten Verfahrens gezeigt, wenn beispielsweise die Kohlenmonoxid enthaltenden Abgase aus vorangehenden Prozessen, insbesondere aus dem Amidierungsschritt, zusammen mit Dampf durch die Veresterungsanlage geleitet werden. Auf diese Weise erfolgt eine nochmalige Vereinigung des Gasgemisches von Verbindungen, die als Feststoff oder als Flüssigkeit abgetrennt werden können. Zum anderen werden diese an einer zentralen Stelle gesammelt und können der weiteren Verwertung oder Entsorgung zugeführt werden.

Das im Rahmen der Veresterung und der anschließenden Vorreinigung erhaltene MMA bzw. der erhaltene Methacrylsäureester oder die erhaltene Methacrylsäure werden anschließend einer weiteren Behandlung zugeführt. Aus der Veresterung resultiert als verbleibender Reststoff verdünnte Schwefelsäure, die ebenfalls einer weiteren Verwertung zugeführt werden kann.

### Vorreinigung des Esters oder der Säure

Im Rahmen des hier vorgestellten Verfahrens kann der Gegenstand der vorliegenden Erfindung auch im Zusammenhang mit einem Verfahren zur Vorreinigung von Methacrylsäure oder Methacrylsäureester eingesetzt werden, wie dieses in dem nachfolgenden Verfahrenselement beschrieben wird. So wird grundsätzlich rohe Methacrylsäure oder ein roher Methacrylsäureester einer weiteren Reinigung unterzogen, um zu einem möglichst reinen Produkt zu gelangen. Eine derartige ein weiteres Verfahrenselement darstellende Reinigung kann beispielsweise einstufig sein. Es hat sich jedoch in vielen Fällen als vorteilhaft herausgestellt, wenn eine solche Reinigung mindestens zwei Stufen umfasst, wobei in einer ersten Vorreinigung, wie sie hier beschrieben wird, die niedrigsiedenden Bestandteile des Produktes entfernt werden. Hierzu wird roher Methacrylsäureester bzw. rohe Methacrylsäure zunächst in eine Destillationskolonne überführt, in welcher die niedrigsiedenden Bestandteile und Wasser abgetrennt werden können. Hierzu wird der rohe Methacrylsäureester einer Destillationskolonne zugeführt, wobei die Zugabe etwa in der oberen Hälfte der Kolonne durchgeführt wird. Der Kolonnensumpf wird mit Dampf beispielsweise derart beheizt, dass eine Wandtemperatur von etwa 50 bis etwa 120 °C erreicht wird. Die Reinigung wird unter Vakuum durchgeführt. Der Druck innerhalb der Kolonne beträgt im Fall des Esters vorzugsweise etwa 100 bis etwa 600 mbar. Der Druck innerhalb der Kolonne beträgt im Fall der Säure vorzugsweise etwa 40 bis etwa 300 mbar.

Am Kolonnenkopf werden die niedrigsiedenden Bestandteile abgenommen. Insbesondere können dieses beispielsweise Ether, Aceton und Methylformiat sein. Die Brüden werden anschließend über einen oder mehrere Wärmetauscher kondensiert. Dabei hat es sich beispielsweise in einigen Fällen bewährt, zunächst eine Kondensation über zwei in Serie geschaltete, mit Wasser gekühlte Wärmetauscher durchzuführen. Es ist jedoch ebenso möglich, an dieser Stelle auch nur einen Wärmetauscher einzusetzen. Die Wärmetauscher werden vorzugsweise zur Erhöhung der Flussgeschwindigkeit und um die Ausbildung stationärer Phasen zu verhindern, in senkrechtem Zustand betrieben. Dem wassergekühlten Wärmetauscher oder den wassergekühlten Wärmetauschern nachgeschaltet, kann ein solegekühlter Wärmetauscher sein, es ist jedoch auch möglich, eine Kaskade von zwei oder mehr solegekühlten Wärmetauschern nachzuschalten. In der Kaskade von Wärmetauschern werden die Brüden kondensiert, mit Stabilisator versehen und beispielsweise einem Phasentrenner zugeführt. Da die Brüden auch Wasser enthalten können, wird eine evtl. anfallende wässrige Phase entsorgt oder einer weiteren Verwertung zugeführt. Als weitere Verwertung bietet sich beispielsweise die Rückführung in eine Veresterungsreaktion, beispielsweise in eine Veresterungsreaktion wie sie oben beschrieben wurde, an. In diesem Fall wird die wässrige Phase vorzugsweise in den ersten Veresterungskessel rückgeführt.

Die abgetrennte organische Phase wird als Rückfluss in den Kolonnenkopf eingespeist. Ein Teil der organischen Phase kann wiederum zum Besprühen der Wärmetauscherköpfe und des Kolonnenkopfes eingesetzt werden. Da es sich bei der abgetrennten organischen Phase um eine Phase handelt, die mit Stabilisator versetzt ist, lässt sich so wirksam einerseits die Ausbildung von Ruhezonen verhindern. Andererseits bewirkt die Gegenwart des Stabilisators eine weitere Unterbindung der Polymerisationsneigung der abgetrennten Brüden.

Der aus den Wärmetauschern erhaltene Kondensatstrom wird darüber hinaus vorzugsweise derart mit demineralisiertem Wasser versetzt, dass im Phasentrenner eine ausreichende Abscheidewirkung erzielt werden kann.

Die nach der Kondensation in der Wärmetauscherkaskade verbleibenden, gasförmigen Verbindungen können, vorzugsweise mittel Dampfstrahler als Unterdruckerzeuger, nochmals über einen oder mehrere weitere Wärmetauscher einer Kondensation unterzogen werden. Es hat sich dabei unter ökonomischen Gesichtspunkten als vorteilhaft herausgestellt, wenn im Rahmen einer solchen Nachkondensation nicht nur die gasförmigen Stoffe aus der Vorreinigung kondensiert werden. So ist es beispielsweise möglich, einer derartigen Nachkondensation weitere gasförmige Stoffe zuzuführen, wie sie sich aus der Hauptreinigung von Methacrylsäureestern ergeben. Der Vorteil einer derartigen Verfahrensweise liegt beispielsweise darin, dass so ein Anteil an Methacrylsäureester, der im Rahmen der Hauptreinigungsstufe nicht kondensiert wurde, im Rahmen der Vorreinigung nochmals über den Phasenabscheider in die Reinigungskolonne überführt werden kann. So wird beispielsweise gewährleistet, dass eine Ausbeutemaximierung stattfinden kann, und möglichst geringe Verluste an Methacrylsäureester auftreten. Außerdem kann durch die geeignete Wahl der Auslegung und des Betriebs dieser weiteren Wärmetauscher die Zusammensetzung des diese Wärmetauscher verlassenden Abgases, insbesondere der Gehalt an Leichtsiedern, eingestellt werden.

Aufgrund der Zuführung von Wasser im Rahmen der Vorreinigung des Methacrylsäureesters kann der Wassergehalt in der Veresterung und die Konzentration an niedrigsiedenden Bestandteilen im Roh-Methylmethacrylat insgesamt kontinuierlich ansteigen. Um dies zu vermeiden, kann es vorteilhaft sein, einen Teil des dem System zugeführten Wassers, vorzugsweise kontinuierlich, aus dem System auszuschleusen. Dieses Ausschleusen kann grundsätzlich beispielsweise in einer Größenordnung erfolgen, in der in der Vorreinigung dem System Wasser zugeführt wird. Die im Phasentrenner abgeschiedene wässrige Phase weist üblicherweise einen Gehalt an organischen Inhaltsstoffen auf. Es kann daher vorteilhaft sein, dieses Wasser einer Form der Entsorgung zuzuführen, die diesen Gehalt an organischen Stoffen ausnutzt.

So kann es beispielsweise vorteilhaft sein, wenn ein derart mit organischen Stoffen belastetes Wasser im Rahmen eines Schwefelsäurespaltverfahrens dem Brennraum zugemischt wird. Aufgrund der oxidierbaren Inhaltsstoffe kann so deren Brennwert, zumindest teilweise, noch genutzt werden. Darüber hinaus wird so eine möglicherweise kostspielige Entsorgung des mit organischen Stoffen belasteten Wassers oft vermieden.

### Feinreinigung des Methacrylsäureesters

Zur Feinreinigung des Methacrylsäureesters wird der rohe, vorgereinigte Methacrylsäureester einer erneuten Destillation unterzogen. Dabei wird der rohe Methacrylsäureester mit Hilfe einer Destillationskolonne von seinen hochsiedenden Bestandteilen befreit und so ein Reinmethacrylsäureester erhalten. Hierzu wird der rohe Methacrylsäureester in einer dem Fachmann bekannten Weise in die untere Hälfte einer Destillationskolonne eingebracht.

Die Destillationskolonne kann grundsätzlich einer beliebigen, dem Fachmann geeignet erscheinenden Ausführung entsprechen. Es hat sich jedoch für die Reinheit des erhaltenen Produkts in vielen Fällen als vorteilhaft herausgestellt, wenn die Destillationskolonne mit einer oder mehreren Packungen betrieben wird, die den folgenden Vorgaben etwa entspricht:

Zum einen sollen sich in den Kolonnen genauso wie in den anderen mit Methacrylsäureester durchströmten Leitungen möglichst wenig so genannte "Toträume" bilden. Die Toträume führen zu einer vergleichsweise langen Verweilzeit der Methacrylsäureester, die deren Polymerisation begünstigen. Dieses führt wiederum zu kostspieligen Produktionsunterbrechungen und Reinigungen der entsprechenden mit Polymer zugesetzten Teile. Der Bildung von Toträumen kann unter anderem dadurch begegnet werden, dass sowohl durch Auslegung als auch durch eine geeignete Betriebsweise der Kolonnen, diese stets mit einer ausreichenden Menge von Flüssigkeit beladen werden, so dass eine ständige Umspülung der Kolonnen und insbesondere der Kolonneneinbauten wie Packungen erreicht wird. So können die Kolonnen Sprüheinrichtungen aus, die zum Besprühen der Kolonneneinbauten ausgelegt sind auf. Weiterhin können die Kolonneneinbauten untereinander oder mit der Kolonne über unterbrochene Haftnähte verbunden sein. Derartige Haftnähte weisen mindestens etwa 2, vorzugsweise mindestens etwa 5 und besonders bevorzugt mindestens etwa 10 Unterbrechungen auf 1 m Haftnahtlänge auf. Die Länge dieser Unterbrechungen kann so gewählt sein, dass diese mindestens etwa 10, vorzugsweise mindestens etwa 20 und besonders bevorzugt mindestens etwa 50 %, im allgemeinen aber nicht mehr als 95 % der Haftnahtlänge ausmachen. Eine andere bauliche Maßnahme kann darin liegen, dass in den Kolonneninnenbereichen, insbesondere die mit der Methacrylsäureester in Kontakt kommen, weniger als etwa 50 %, vorzugsweise weniger als etwa 25 % und besonders bevorzugt weniger als etwa 10 % aller Flächen, insbesondere von Kolonneneinbauten, horizontal verlaufen. So können beispielsweise die in das Innere der Kolonne mündenden Stutzen konisch bzw. mit schrägen Flächen ausgestaltet sein. Ferner kann eine Maßnahme darin bestehen, die während des Betriebs der Kolonne im Kolonnensumpf befindliche Menge flüssiger von Methacrylsäureester so gering wie möglich zu halten und anderseits eine Überhitzung dieser Menge trotz moderaten Temperaturen und großen Verdampfungsflächen während des Verdampfen zu vermeiden. Hierbei kann es vorteilhaft sein, dass die Flüssigkeitsmenge im Kolonnensumpf im Bereich von etwa 0,1 bis 15 % und vorzugsweise etwa 1 bis 10 % der Gesamtmenge an Methacrylsäureester in der Kolonne ausmacht. Die in diesem Abschnitt vorgeschlagenen Maßnahmen können auch bei der Destillation von Methacrylsäure Anwendung finden.

Im Rahmen der Reinigung des Methacrylsäureesters werden dessen hochsiedende Bestandteile durch Destillation vom Produkt getrennt. Hierzu wird der Kolonnensumpf mit Dampf beheizt. Die Sumpftemperatur beträgt dabei vorzugsweise etwa 50 bis etwa 80 °C, insbesondere etwa 60 bis etwa 75 °C bei Wandtemperatur von weniger als etwa 120 °C.

Das im Kolonnensumpf anfallende Material wird vorzugsweise kontinuierlich abgeführt und über einen Wärmetauscher oder eine Kaskade von mehreren Wärmetauschern auf eine Temperatur in einem Bereich von etwa 40 bis etwa 80 °C, vorzugsweise etwa 40 bis etwa 60 °C und besonders bevorzugt in einem Bereich von etwa 50 bis 60 °C abgekühlt.

Dieses Material, das überwiegend Methacrylsäureester, Hydroxyisobuttersäureester, Methacrylsäure und Stabilisatorkomponenten enthält, wird anschließend über einen Lagerbehälter, beispielsweise entsorgt oder einer anderweitigen Verwendung zugeführt. Es hat sich in vielen Fällen als vorteilhaft erwiesen, wenn das im Kolonnensumpf gewonnene Material in die Veresterungsreaktion zurückgeführt wird. Beispielsweise wird dabei das Material aus dem Kolonnensumpf in den ersten Veresterungskessel rückgeführt. Daraus ergibt sich der Vorteil, dass im Hinblick auf eine möglichst wirtschaftliche Fahrweise und eine möglichst hohe Ausbeute im Kolonnensumpf enthaltene, höhersiedende Verbindungen in die Veresterungsreaktion zurückgeführt werden.

Am Kolonnenkopf wird der destillativ aufgereinigte Methacrylsäureester entnommen und über einen Wärmetauscher oder eine Kaskade von zwei oder mehr Wärmetauschern gekühlt. Dabei kann die Wärme der Brüden durch wassergekühlte Wärmetauscher oder durch solegekühlte Wärmetauscher oder durch eine Kombination aus beidem abgeführt werden. Es hat sich in einigen Fällen bewährt, wenn die Brüden aus der Destillationskolonne in zwei oder mehr parallel geschaltete Wärmetauscher überführt werden, die mittels Wasserkühlung betrieben werden. Die nicht kondensierten Anteile aus den wassergekühlten Wärmetauschern können beispielsweise in einen solegekühlten Wärmetauscher oder eine Kaskade von zwei oder mehr solegekühlten Wärmetauschern eingeleitet werden, die seriell oder parallel angeordnet sein können. Die aus den Wärmetauschern erhältlichen Kondensate werden in einen Sammelbehälter eingeleitet und mittels einer Pumpe über einen weiteren Wärmetauscher oder eine Kaskade von zwei oder mehr weiteren Wärmetauschern einem Pufferbehälter zugeführt. Der Kondensatstrom wird dabei beispielsweise über eine Kaskade von einem oder zwei wassergekühlten Wärmetauschern und einem oder zwei solegekühlten Wärmetauschern auf eine Temperatur in einem Bereich von etwa 0 bis etwa 20 °C, vorzugsweise etwa 0 bis etwa 15 °C und besonders bevorzugt in einem Bereich von etwa 2 bis 10 °C heruntergekühlt.

Dem Kondensatstrom wird ein Teilstrom entnommen, der über den Kolonnenkopf in die Destillationskolonne zurückgeführt wird. Die Einspeisung des Kondensatstromes in den Kolonnenkopf kann dabei grundsätzlich auf beliebige Weise z.B. über Verteiler erfolgen. Es kann jedoch vorteilhaft sein, wenn ein Teil des Kondensatstromes oberhalb des Kolonnenkopfes in die Brüdenleitung eingespeist, beispielsweise eingesprüht, wird. Weiterhin ist es bevorzugt, dass mit dieser Einspeisung Stabilisator in den Kolonnenkopf eingetragen wird.

Ein weiterer Teilstrom des zur Rückführung in die Kolonne vorgesehenen Kondensates kann beispielsweise vor Einbringung in die Brüdenleitung abgezweigt und direkt in den Kolonnenkopf eingebracht werden. Auch hier ist es bevorzugt, dass mit dieser Einspeisung Stabilisator in den Kolonnenkopf eingetragen wird. Die Einbringung in den Kolonnenkopf kann dabei beispielsweise dergestalt geschehen, dass das Innere des Kolonnenkopfes mit dem Kondensat so besprüht wird, dass sich keine Ruhezonen im Kolonnenkopf ausbilden können, an denen eine Polymerisation des Methacrylsäureesters erfolgen kann. Es kann darüber hinaus vorteilhaft sein, wenn einem Kondensatteilstrom, der in die Kolonne zurückgeführt wird, ein Stabilisator zur Verhinderung von Polymerisation zugefügt wird. Dies kann beispielsweise dadurch geschehen, dass dem zum Besprühen des Kolonnenkopfes vorgesehenen Kondensatteilstrom eine entsprechende Menge an Polymerisationsinhibitor als Stabilisator zugefügt wird. Dabei hat es sich in einigen Fällen als vorteilhaft erwiesen, wenn der Kondensatteilstrom nach der Zugabe des Stabilisators, aber vor dem Eintritt in den Kolonnenkopf eine geeignete Mischvorrichtung, vorzugsweise einen Statikmischer durchläuft, um eine möglichst uniforme Verteilung des Stabilisators im Kondensatteilstrom zu erzielen.

Die im Rahmen des Reinigungsverfahrens anfallenden, nicht kondensierbaren gasförmigen Stoffe werden beispielsweise der Entsorgung zugeführt.

Das im Pufferbehälter befindliche Rohprodukt wird mit Hilfe eines Solekühlers auf einer Temperatur von etwa 0 bis etwa 20 °C, vorzugsweise etwa 0 bis etwa 15 °C und besonders bevorzugt in einem Bereich von etwa 2 bis 10 °C gehalten.

Um ggf. weitere Verunreinigungen aus dem Produkt zu entfernen und zur Reinstmethacrylsäureestern zu gelangen, kann das Produkt noch einer adsorptiven Reinigungsstufe unterzogen werden. Es hat sich dabei beispielsweise bewährt, wenn das Reinprodukt ganz oder zumindest ein Teil des Reinproduktes mit Hilfe eines Molekularsiebes weiter gereinigt wird. Besonders saure Verunreinigungen, insbesondere im Rahmen des Herstellungsverfahren gebildete Ameisensäure, kann so auf einfache Weise aus dem Produktstrom entfernt werden. Dabei hat es sich darüber hinaus in einigen Fällen bewährt, wenn der Produktstrom nach dem Durchlaufen der adsorptiven Reinigungsstufe noch einen oder mehrere Filter durchläuft, um ggf. im Produkt enthaltene Feststoffe zu entfernen.

Die im Rahmen der Aufarbeitung anfallenden Stoffströme umfassen überwiegend polymerisierbare Verbindungen. Um, wie bereits im Rahmen dieses Textes mehrfach beschrieben, die Ausbildung von Ruhezonen zu unterbinden, hat es sich auch im Fall des hier beschriebenen Verfahrens als vorteilhaft herausgestellt, wenn die Teile der Anlage, die mit Methacrylsäureester in Berührung kommen, ständig mit Methacrylsäureester überströmt sind. Im Rahmen einer weiteren Ausführungsform des hier vorgestellten Verfahrens wird daher ein Teilstrom an Methacrylsäureester nach dem Pufferbehälter, jedoch vor der adsorptiven Reinigungsstufe entnommen, um die Kopfbereiche derjenigen Wärmetauscher zu überspülen, welche die aus der Destillationskolonne stammenden Brüden aufnehmen.

Das im Rahmen der Reinigungsstufe gewonnene Produkt wird anschließend mit einer Temperatur in einem Bereich von etwa -5 bis etwa 20 °C, vorzugsweise etwa 0 bis etwa 15 °C und besonders bevorzugt in einem Bereich von etwa 2 bis 10 °C der Reinigungsstufe entnommen.

### Strippen der verbrauchten Säure

Im Rahmen des hier vorgestellten Verfahrens kann es beispielsweise in einem weiteren Verfahrenselement sinnvoll sein, die bei dem Verfahren anfallende, verbrauchte Schwefelsäure einer Reinigung zu unterziehen, um sie anschließend wieder in das Verfahren zurückzuführen. Dabei kann beispielsweise ein Strom mit verbrauchter Schwefelsäure, wie er sich aus der Veresterung erhalten lässt, in einem Flotationsbehälter mit Dampf beaufschlagt werden. Dabei kann sich zumindest ein Teil der enthaltenen Feststoffe auf der Oberfläche der Flüssigkeit abscheiden, wobei diese abgeschiedenen Feststoffe ausgekreist werden können. Die Brüden werden anschließend in einem Wärmetauscher, vorzugsweise mit Wasserkühlung, kondensiert, gekühlt und in die Veresterungsreaktion zurückgeführt.

Es hat sich dabei als in einigen Fällen als vorteilhaft erwiesen, wenn zur Verminderung von Korrosion in den Wärmetauschern und zur weiteren Verbesserung der Kühlwirkung ein Gemisch aus Wasser und organischen Verbindungen, wie es im Rahmen der Veresterung bei der Reinigung des hergestellten Methacrylsäureesters durch Waschen erhalten wird, in die Wärmetauscher derart eingebracht wird, dass die Köpfe der Wärmetauscher mit diesem Gemisch besprüht werden. Neben der korrosionsvermindernden Wirkung und der Kühlung der Säure im Wärmetauscher weist diese Vorgehensweise einen weiteren Vorteil auf. Material, das aus der Veresterung stammt (ein Gemisch aus Wasser und überwiegend Methanol), wird zusammen mit der aus eben diesem Prozess stammenden Methacrylsäure und Methacrylsäureester in den Veresterungsprozess zurückgeführt. Im Stripper werden durch die oben beschriebene Flotation Gemische von Säure und Feststoffen erhalten. Diese werden nach ihrer Abtrennung einer beliebigen weiteren Verwendung oder der Entsorgung zugeführt. Möglich ist beispielsweise, das erhaltene Gemisch in einer Spaltanlage zu verbrennen und damit wieder Schwefelsäure zu erzeugen.

Die beim Strippen anfallenden, nicht kondensierbaren gasförmigen Verbindungen werden einer beliebigen Weiterverwendung zugeführt oder entsorgt.

Die hier beschriebene Anlage zur Entfernung von Feststoffen aus der verbrauchten Säure sowie zur Rückführung von Material aus dem Veresterungsprozess in eben diesen Prozess kann aus Gründen der Betriebssicherheit beispielsweise auch doppelt ausgeführt sein. So können die zwei oder mehr Flotationsbehälter zeitlich versetzt eingesetzt werden. Da sich in diesen Behältern Feststoffe absetzen können, ist es vorteilhaft, diese zu entfernen, wenn der jeweilige Flotationsbehälter sich nicht im Einsatz befindet.

Weiterhin betrifft die vorliegende Erfindung die Verwendung der durch das erfindungsgemäße Verfahren erhältlichen Methacrylsäure beziehungsweise des durch das erfindungsgemäße Verfahren erhältlichen Methacrylsäureester in Fasern, Filmen, Lacken, Formmassen, Formkörpern, Papierhilfsmittel, Lederhilfsmittel, Flockungsmittel und Bohrzusätze. Zudem betrifft die vorliegende Erfindung Fasern, Filme, Lacke, Formmassen, Formkörper, Papierhilfsmittel, Lederhilfsmittel, Flockungsmittel und Bohrzusätze, die auf einer durch das erfindungsgemäße Verfahren erhältlichen Methacrylsäure beziehungsweise einem durch das erfindungsgemäße Verfahren erhältlichen Methacrylsäureester basieren.

Das Vorstehende wird nunmehr anhand von Zeichnungen beispielhaft erläutert. Es zeigen:
- Fig. 1:: einen Anlagenverbund zur Herstellung und Verarbeitung von mit Methacrylsäure bzw. Methylmethacrylat,
- Fig. 2:: schematisch eine Anlage zur Herstellung von Acetoncyanhydrin,
- Fig. 3:: schematisch eine Aufarbeitungsanlage von Acetoncyanhydrin,
- Fig. 4:: schematisch Amidierungsanlage,
- Fig. 5:: schematisch eine Veresterungsanlage,
- Fig. 6:: schematisch eine Anlage zur Vorreinigung des Esters,
- Fig. 7:: die Feinreinigungsanlage des Esters.

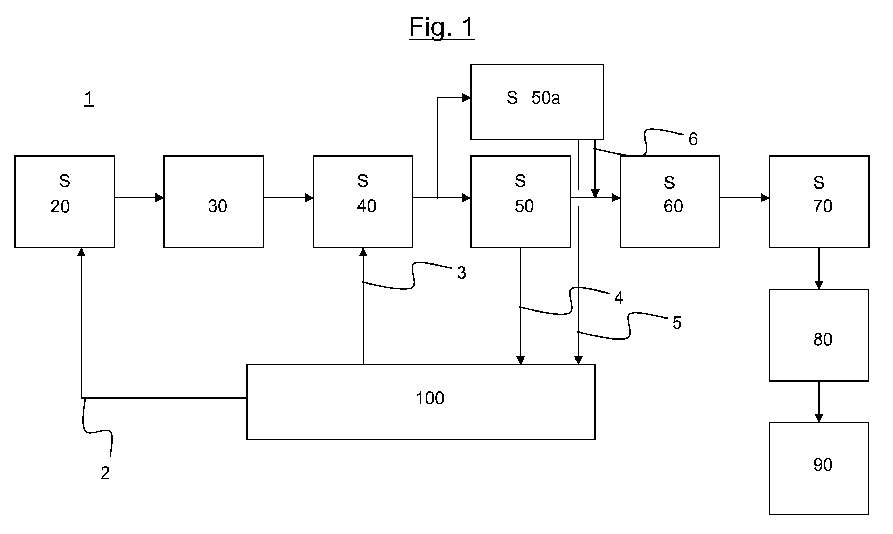

In Fig. 1 sind die bevorzugten Elemente einen Anlagenverbund 1 zur Herstellung von Methacrylsäure bzw. Methacrylsäureestern und deren Weiterverarbeitungsprodukten gezeigt. Der Anlagenverbund 1 weist verschiedene untereinander meist fluidleitenden verbundene Anlagen als Elemente dieses Verbunds auf. Zu diesem Anlagenverbund gehört die Acetoncyanhydrinherstellung 20, gefolgt von der Acetoncyanhydrinaufarbeitung 30, gefolgt von einer Amidierung 40, gefolgt von einer Veresterung/Hydrolyse 50/50a), gefolgt von einer Aufarbeitung für Ester oder Methacrylsäure 60 wiederum gefolgt von einer Feinreinigung 70, nach der der Ester, meist Methylmethacrylat, oder Methacrylsäure vorliegt. Der so gewonnene Reinester/Reinsäure kann einer Weiterverarbeitungsanlage 80 zugeführt werden. Als Weiterverarbeitungsanlagen 80 kommen vor allem Polymerisationsvorrichtungen und Reaktoren für weitere organische Reaktionen in Betracht. In den Polymerisationsreaktoren können Polymethacrylate hergestellt werden und in den Reaktoren für organische Reaktionen können die hier gewonnenen reinen Monomere zu weiteren organischen Verbindungen umgesetzt werden. Auf die Weiterverarbeitungsanlage oder die Weiterverarbeitungsanlagen 80 folgt eine Konfektionierung 90. Sofern es sich bei den Weiterverarbeitungsprodukten um Polymere aus der Methacrylsäure oder Methacrylsäureester, insbesondere Methylmethacrylat, handelt werden diese zu Fasern, Formmassen, insbesondere Granulat, Folien, Platten, Automobilteile und anderen Formkörpern durch geeignete Geräte wie Extruder, Blasextruder, Spritzgussgeräte, Spinndüsen und dergleichen, weiterverarbeitet. Weiterhin beinhaltet der Anlagenverbund 1 in vielen Fällen eine Schwefelsäureanlage 100. Hierbei kommen grundsätzlich alle dem Fachmann hierfür geeignet erscheinenden Schwefelsäureanlagen in Betracht. Beispielsweise sei auf die in Kapitel 4, Seite 89 ff. in "Integrated Pollution Prevention and Control -Draft Reference Document on Best Avalible Techniques for the Manufacture of Large Volume Inorganic Chemicals - Amonia Acids and Fertelizers", erhältlich über die Europäische Kommission, in diesem Zusammenhang verwiesen. Die Schwefelsäureanlage 100 ist mit einer Reihe anderer Anlagen verbunden. So wird die Acetoncyanhydrinherstellung 20 über eine Schwefelsäureleitung 2 mit konzentrierter Schwefelsäure versorgt. Außerdem besteht eine weitere Schwefelsäureleitung 3 zwischen der Schwefelsäureanlage 100 und der Amidierung 40. Die auch als "Spent Acid" bezeichnete verdünnte Schwefelsäure aus der Veresterung 50 (Hydrolyse 50a) wird zu der Schwefelsäureanlage 100 durch die Leitungen für verbrauchte Schwefelsäure 4 bzw. 5 überführt. In der Schwefelsäureanlage 100 kann die verdünnte Schwefelsäure aufgearbeitet werden. Die Aufarbeitung der verdünnten Schwefelsäure kann beispielsweise wie in WO 02/23088 A1 oder WO 02/23089 A1 beschrieben erfolgen. Im Allgemeinen sind die Anlagen aus dem Fachmann geläufigen und für die jeweiligen Beanspruchungen geeignet erscheinenden Materialien ausgeführt. Meist handelt es sich hierbei um Edelstahl, der insbesondere eine besondere Säurefestigkeit besitzen muss. Die Bereiche der Anlagen, die mit Schwefelsäure und insbesondere mit konzentrierter Schwefelsäure betrieben werden, sind darüber hinaus mit keramischen Materialien oder Kunststoffen ausgekleidet und geschützt. Zudem kann die in der Methacrylsäureanlage 50a erhaltene Methacrylsäure über eine Methacrylsäureleitung 6 der Vorreinigung 60 zugeführt werden. Weiterhin hat es sich bewährt, dass in der Acetoncyanhydrinherstellung 20, der Amidierung 40, der Veresterung 50, der Hydrolyse 50a, der Vorreinigung 60 und auch der Endreinigung 70 einen mit "S" gekennzeichneten Stabilisators zuzusetzen.

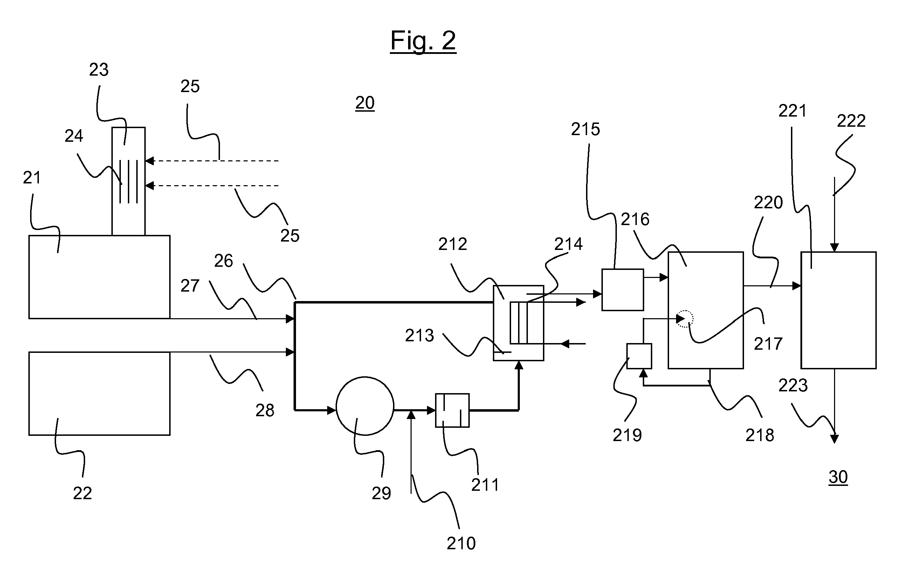

In der in Figur 2 dargestellten Acetoncyanhydrinherstellung 20 wird das Aceton in einem Acetonbehälter 21 und die Blausäure in einem Blausäurebehälter 22 bereitgestellt. Der Acetonbehälter 21 weist einen Waschturm 23 auf, der in seinem oberen Bereich einen oder mehrere Kühlelemente 24 aufweist. In den Waschturm 23 mündet eine Reihe von Abgasleitungen 25, die aus verschiedenen Anlagen des Anlagenverbunds 1 stammen. In einen Schlaufenreaktor 26 wird das Aceton über die Acetonzuleitung 27 und die Blausäure über die Blausäurezuleitung 28 eingespeist stromab der Blausäurezuleitung 28 befindet sich eine Pumpe 29, wiederum gefolgt von einer Katalysatoreinspeisung 210, auf die ein statischer Mischer 211 folgt. Hiernach schließt sich ein Wärmetauscher 212 an, der eine Reihe von Strömungswiderständen 213 und mindestens eine Kühlleitung 214 aufweist. In dem Schlaufenreaktor 26 wird das aus Aceton, Blausäure und Katalysator bestehende Reaktionsgemisch zu einem erheblichen Teil in einem Kreislauf gefahren, der durch fette Linien gekennzeichnet ist. Aus dem Wärmetauscher 212 wird die Reaktionsmischung über die Strömungswiderstände entlang der Kühlleitungen 214 geführt und ein Teil des Kreislaufstromes in einen weiteren Wärmetauscher 215 geleitet, an den sich ein Sammelbehälter 216 anschließt, in dem sich eine Düse 217 als Teil eines Kühlkreislaufs 218 mit einem Wärmetauscher 219 befindet, wodurch das Reaktionsprodukt zum einen in Bewegung und zum anderen kühl gehalten wird. Über eine sich an den Sammelbehälter 216 anschließende Ableitung 220 ist ein Stabilisierbehälter 221 angeschlossen, an den eine Schwefelsäurezuleitung 222 mündet und aus dem das Rohacetoncyanhydrin durch die Ableitung 223 in die Acetoncyanhydrinaufarbeitung 30 geführt wird.

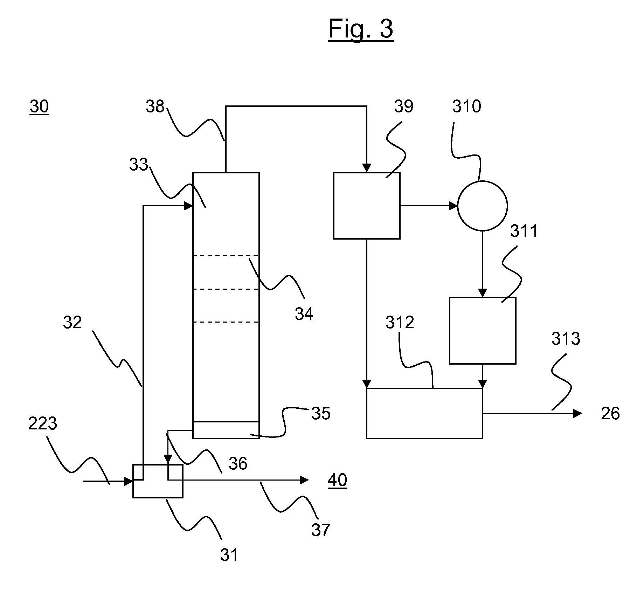

In Figur 3 mündet aus der Cyanhydrinherstellung 20 kommend die Ableitung 223 in einen Wärmetauscher 31, in dem der aus der Cyanhydrinherstellung 20 kommende Strom erwärmt wird. An den Wärmetauscher 31 schließt sich eine Brüdenzuleitung 32 an, die in dem oberen, vorzugsweise dem Kopfbereich einer Kolonne 33 mündet. Die Kolonne 33 weist eine Vielzahl von Packungen 34 auf, die meist als Böden ausgestaltet sind. Im unteren Bereich der Kolonne 33 befindet sich der Kolonnensumpf 35 aus dem eine Sumpfableitung 36 in den Wärmeaustauscher 31 führt und den über die Ableitung 233 in den Wärmeaustausch 31 geführten Ströme erwärmt. An den Wärmetauscher 31 schließt sich eine Reinproduktführung 37 an, auf die die Amidierung 40 stromab folgt. Im Kopfbereich der Kolonne 33 befindet sich eine Kopfableitung 38, die in einen Wärmetauscher 39 mündet, an den sich eine Vakuumpumpe 310 anschließt, die wiederum in einen Wärmetauscher 311 mündet. Sowohl der Wärmetauscher 39 als auch der Wärmetauscher 311 sind über Leitungen mit einem Kühlbehälter 312 verbunden an den sich eine Rückführung 313 anschließt, die mit dem Schlaufenreaktor 26 in der Acetoncyanhydrinherstellung 20 verbunden ist.

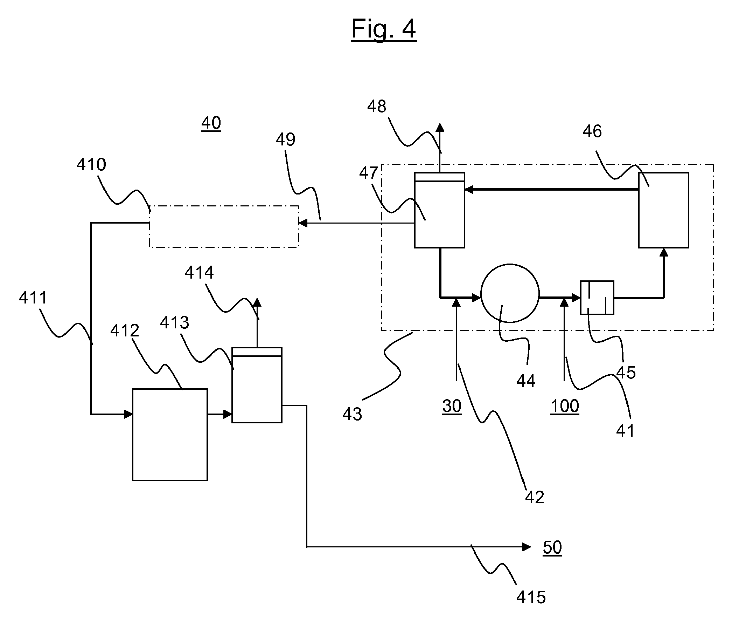

Die in Fig. 4 abgebildete Amidierung 40 weist zunächst eine Acetoncyanhydrinzuführung 41 und eine Schwefelsäurezuführung 42 auf, die in einen Schlaufenreaktor 43 münden. Die mit der Acetoncyanhydrinaufarbeitung 30 verbundene Acetoncyanhydrinzuführung 41 mündet in den Kreislauf des Schlaufenreaktors 43 nach einer Pumpe 44 vor einem Mischer 45. Vor dieser Pumpe 44 mündet die Schwefelsäurezuführung 42. Stromab des Mischers 45 folgt ein Wärmeaustauscher 46 der wiederum in einen Gasabscheider 47 mündet aus dem zum einen eine Gasableitung 48 und eine Zuleitung 49 zu einem weiteren Schlaufenreaktor 410 abgeht. Der weitere Schlaufenreaktor 410 oder ein Dritter ist vergleichbar wie der erste Schlaufenreaktor 43 aufgebaut. Von dem weiteren Schlaufenreaktor 410 geht eine Zuleitung 411 in einen Wärmeaustauscher 412, auf den ein Gasabscheider 413 folgt, von dem zu einem eine Gasableitung 414 und eine Amidleitung 415 abgeht, die zu der Veresterung/Verseifung 50 /MAS-Anlage 50a führt.

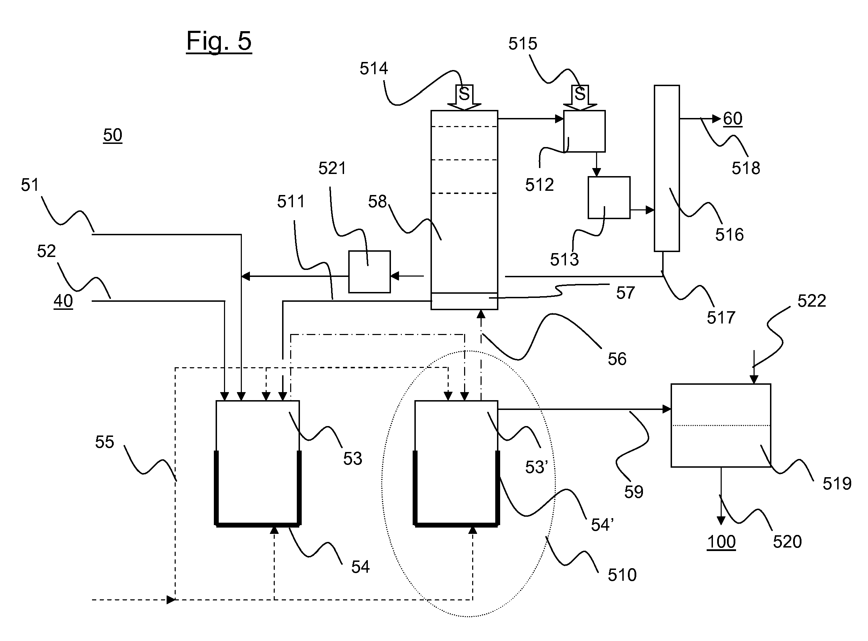

Die Figur 5 zeigt die Veresterung 50, in der eine Wasser und organische Lösungsmittel führende Lösungsmittelleitung 51 und eine mit der Amidierung 40 verbundene Amidleitung 52 in einen Kessel 53 münden, der durch eine Kesselheizung 54 beheizbar ist. In den Kessel 53 mündet weiterhin eine gestrichelt gezeichnete Alkoholleitung 55. Die Alkoholleitung 55 mündet sowohl in dem oberen als auch in dem unteren Bereich des Kessels 53. Über eine mit Strichpunkt gekennzeichnete Esterbrüdenleitung 56 ist der erste Kessel 53 mit einem weiteren Kessel 53' verbunden, der eine weitere Kesselheizung 54' aufweist. Auch dieser weitere Kessel 53' ist sowohl von unten als auch von oben mit der Alkoholleitung 55 verbunden. An den oberen Bereich des Kessels 53' schließt sich die Esterbrüdenleitung 56 an, die in einen Sumpf 57 einer Kolonne 58 mündet. Weiterhin befindet sich im oberen Bereich des Kessels 53' eine Leitung für verdünnte Schwefelsäure 59. Eine in der gepunkteten Ellipse eingerahmte Kesseleinheit 510 wird aus einem beheizbaren Kessel 53 und 54 mit Alkoholleitung 55 und Esterbrüdenleitung 56 gebildet. Es können ein, zwei oder mehr derartiger Kesseleinheiten kaskadenartig aufeinander folgen, wobei jede dieser Kesseleinheiten 510 über die Esterbrüdenleitung 56 mit dem Sumpf 57 der Kolonne 58 verbunden ist. Aus dem Sumpf 57 der Kolonne 58 führt weiterhin eine Schwersiederleitung 511 zum Kessel 53, um Wasser und organische Lösungsmittel wieder der Veresterung zuzuführen. Im Oberbereich, vorzugsweise dem Kopf, der Kolonne 58, schließt sich über eine geeignete Leitung ein erster Wärmetauscher 512 gefolgt von einem weiteren Phasentrenner 513 an. Sowohl am Kopf der Kolonne 58 als auch in dem ersten Wärmetauscher 512 können eine erste Stabilisatorzuführung 414 (Stabilisator mit "S" gekennzeichnet) sowie eine weitere Stabilisatorzuführung 515 vorgesehen sein, um einen eine unerwünschte Polymerisation verhindernden Inhibitor bzw. Stabilisator zuzuführen. An den weiteren Phasentrenner 513 schließt sich ein Wäscher 516 an, in dessen unteren Bereich eine Lösemittelleitung 517 abgeht, die über einen Wärmetauscher 521 in der Lösungsmittelleitung 51 mündet. Von dem oberen Bereich des Wäschers 516 geht eine Rohesterleitung ab, die in die Esteraufarbeitung 60 mündet. Die aus dem oberen Bereich des Kessels 53' bzw. des Kessels der letzten Kesseleinheit 510 abgehende Spendsäureleitung 59 mündet in einen Flotationsbehälter 519 zum Abtrennen der Feststoffe bzw. in der Spendsäure nicht löslichen Bestandteile. Aus dem Flotationsbehälter 519 geht eine Spentsäureableitung 520 in die Schwefelsäureanlage 100 sowie eine, die niedriegsiedenden Bestandteile führende, Niedersiederbrüdenleitung 522 zur weiteren Aufarbeitung und Rückführung in die Veresterung.

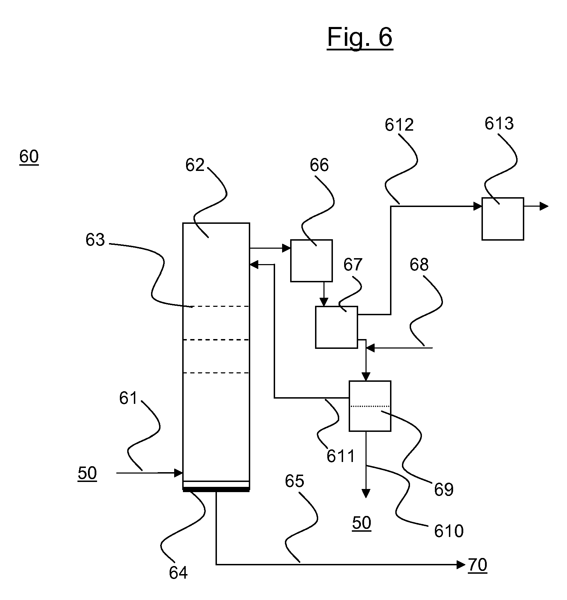

Die in Figur 6 gezeigt Esteraufarbeitung schließt sich über eine Rohesterleitung 61 an die Veresterung 50 an, wobei die Rohesterzuleitung 61 in den mittleren Bereich einer Vakuumdestillationskolonne 62 mündet. Diese Kolonne 62 weist Kolonneneinbauten 63 und eine im unteren Bereich der Kolonne 62 angeordnete Sumpfheizung 64 auf. Von dem unteren Bereich der Kolonne 62, der den Sumpf dieser Kolonne darstellt, geht eine Esterableitung 65 ab, die in die Esterfeinreinigung 70 mündet und den von Leichtsiedern befreiten Rohester somit der Feinreinigung zuführt. Im oberen Bereich der Kolonne 62, meist im Kopf, schließt sich über eine Ableitung ein erster Wärmetauscher 66 und ein weiterer oder mehrere Wärmetauscher 67 an, auf den ein Phasentrenner 69 folgt. In dem Phasentrenner 69 wird die aus dem Wärmetauscher 67 stammende Mischung in organische und wässrige Bestandteile aufgeteilt, wobei sich an den Phasentrenner 69 eine Rückführung 611 im oberen Bereich anschließt, die im oberen Bereich der Kolonne 62 einmündet. Im unteren Bereich des Abscheiders befindet sich eine Wasserableitung 610, die in die Veresterung 50 mündet, um das abgetrennte Wasser der Veresterung wieder zuzuführen. An die Wärmetauscher 66 und 67 schließt sich über eine Unterdruckleitung 612 eine Unterdruckerzeuger 613 an.

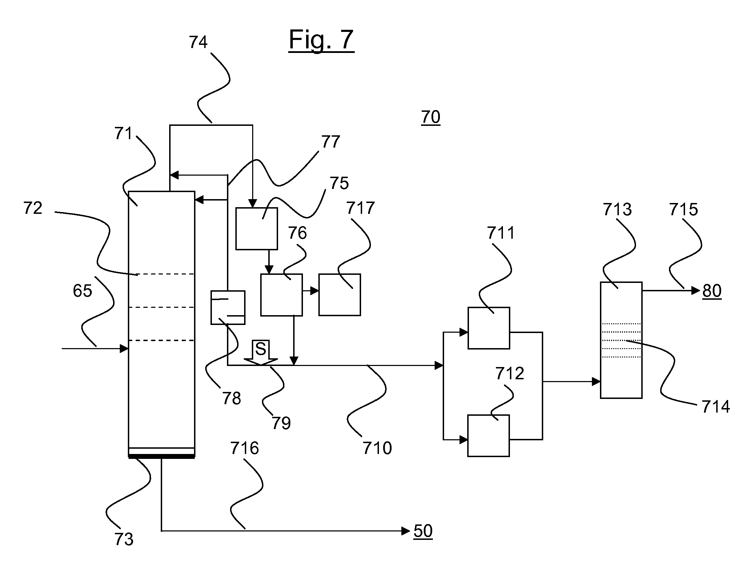

In Figur 7 mündet die aus der Esteraufarbeitung 60 stammende Esterableitung 65 in eine Destillationskolonne 71. Diese umfasst mehrere Kolonneneinbauten 71 sowie im unteren Bereich der Destillationskolonne 71 eine Kolonnensumpfheizung 73. Vom Kopfbereich der Destillationskolonne 71 geht eine Reinesterbrüdenleitung 74 in einen ersten Wärmetauscher 75, auf den ein (oder mehrere) weiterer Wärmetauscher 76 folgen, die mit einem Unterdruckerzeuger 717 verbunden sind. Der Ausgang des weiteren Wärmetauschers 76 weist eine Leitung auf, von der zum einen eine Esterrückführung 77 in den oberen Bereich der bzw. in den Kopf der Destillationskolonne 71 mündet. Die Esterrückführung 77 weist eine Stabilisatorzudosierung 79 auf, die in der Esterrückführung 77 vor einem Mischer 78 angeordnet ist. Zum anderen geht von der Leitung des weiteren Wärmetauschers 76 eine Reinesterableitung 710 ab. An diese schließt sich in Reihenschaltung ein zusätzlicher Wärmetauscher 711 und ein anderer Wärmetauscher 712 an. Auf diese folgt ein Molekularsiebbehälter 713, der Molekularsiebpackungen 714 aufweist. Durch das Molekularsieb weiter gereinigt, wird der Reinstester durch die sich an den Molekularsiebbehälter anschließende Reinstesterableitung in die Weiterverarbeitungsanlage 80 überführt.

### Bezugszeichenliste

- 1: Anlagenverbund
- 2: Schwefelsäureleitung
- 3: weitere Schwefelsäureleitung
- 4: verbrauchte Schwefelsäureleitung - Ester
- 5: verbrauchte Schwefelsäureleitung - Säure
- 6: Methacrysäureleitung
- 20: Acetoncyanhydrinherstellung
- 30: Acetoncyanhydrinaufarbeitung
- 40: Amidierung
- 50: Veresterung
- 50a: Hydrolyse
- 60: Vorreinigung
- 70: Endreinigung
- 80: Weiterverarbeitungsanlage
- 90: Konfektionierung
- 100: Schwefelsäureanlage
- 21: Acetonbehälter
- 22: Blausäurebehälter
- 23: Waschturm
- 24: Kühlelemente
- 25: Abgasleitungen
- 26: Schlaufenreaktor
- 27: Acetonzuleitung
- 28: Blausäurezuleitung
- 29: Pumpe
- 210: Katalysatorzuleitung
- 211: Mischer
- 212: Wärmetauscher
- 213: Strömungswiderstand
- 214: Kühlleitungen
- 215: Wärmetauscher
- 216: Sammelbehälter
- 217: Düse
- 218: Kühlkreislauf
- 219: Wärmetauscher
- 220: Ableitung
- 221: Stabilisierbehälter
- 222: Schwefelsäurezuleitung
- 223: Ableitung
- 31: Wärmetauscher
- 32: Brüdenzuleitung
- 33: Kolonne
- 34: Packungen
- 35: Kolonnensumpf mit Wärmetauscher
- 36: Sumpfableitung
- 37: Reinproduktführung
- 38: Kopfableitung
- 39: Wärmetauscher
- 310: Vakuumpumpe
- 311: Wärmetauscher
- 312: Kühlbehälter
- 313: Rückführung
- 41: Acetoncyanhydrinzuführung
- 42: Schwefelsäurezuführung
- 43: Schlaufenreaktor
- 44: Pumpe
- 45: Mischer
- 46: Wärmetauscher
- 47: Gasabscheider
- 48: Gasableitung
- 49: Zuleitung
- 410: weiterer Schlaufenreaktor
- 411: Zuleitung
- 412: Wärmetauscher
- 413: Gasabscheider
- 414: Gasableitung
- 415: Amidleitung
- 51: Lösungsmittelleitung
- 52: Amidleitung
- 53: erster Kessel
- 54: erste Kesselheizung
- 53': weiterer Kessel
- 54': weitere Kesselheizung
- 55: Alkoholleitung
- 56: Esterbrüdenleitung
- 57: Kolonnensumpf
- 58: Kolonne
- 59: Spendsäureleitung
- 510: Kesseleinheit
- 511: Schwersiederleitung
- 512: Wärmetauscher
- 513: Phasentrenner
- 514: Stabilisatorzuführung
- 515: weitere Stabilisatorzuführung
- 516: Extraktionskolonne
- 517: Lösemittelleitung
- 518: Rohesterleitung
- 519: Flotationsbehälter
- 520: Spentsäureableitung
- 521: Wärmetauscher
- 522: Niedersiederbrüdenleitung
- 61: Rohesterzuleitung
- 62: Vakuumdestillationskolonne
- 63: Kolonneneinbauten
- 64: Sumpfheizung
- 65: Esterableitung
- 66: Wärmetauscher
- 67: Wärmetauscher
- 68: Wasserzufuhr
- 69: Phasentrenner
- 610: Wasserableitung
- 611: Rückführung
- 612: Unterdruckleitung
- 613: Unterdruckerzeuger
- 71: Destillationskolonne
- 72: Kolonneneinbauten
- 73: Kolonnensumpfheizung
- 74: Reinesterbrüdenleitung
- 75: erster Wärmetauscher
- 76: weitere Wärmetauscher
- 77: Esterrückführung
- 78: Mischer
- 79: Stabilisatorzudosierung
- 710: Reinesterableitung
- 711: zusätzlicher Wärmetauscher
- 712: andere Wärmetauscher
- 713: Molekularsiebbehälter
- 714: Molekularsiebpackungen
- 715: Reinstesterableitung
- 716: Schwersiederleitung
- 717: Leichtsiederabzug

## Patentansprüche

1. Verfahren zur Herstellung von Acetoncyanhydrin, bei dem ein Reaktionsgemisch enthaltend Aceton und Blausäure in Gegenwart eines Katalysators in einem Schlaufenreaktor unter Bildung von Acetoncyanhydrin als Reaktionsprodukt zur Reaktion gebracht werden, wobei der Schlaufenreaktor mindestens eine Vorrichtung zum Kühlen des Reaktionsgemischs, mindestens eine Pumpe, mindestens eine Vorrichtung zum Vermischen des Reaktionsgemischs und jeweils mindestens eine Zufuhröffnung zum Zuführen von Blausäure, Aceton und Katalysator aufweist, wobei die Zufuhr des Katalysators stromabwärts von der Zufuhr von Aceton und Blausäure erfolgt.

2. Verfahren nach Anspruch 1 **dadurch gekennzeichnet, dass** die Zufuhr des Katalysators an einer Stelle des Schlaufenreaktors erfolgt, die näher an einer Eintrittsöffnung des Reaktionsgemischs in mindestens eine Vorrichtung zum Vermischen des Reaktionsgemischs liegt als an einer Zufuhröffnung zum Zuführen von Blausäure oder einer Zufuhröffnung zum Zuführen von Aceton oder beiden Zufuhröffnungen.

3. Verfahren nach einem der vorstehenden Ansprüche, **dadurch gekennzeichnet, dass** die Zufuhr des Katalysators an einer Stelle des Schlaufenreaktors erfolgt, an welcher der Weg des Reaktionsgemischs vor Eintritt in eine Eintrittsöffnung des Reaktionsgemischs in mindestens eine Vorrichtung zum Vermischen des Reaktionsgemischs höchstens 30 % des Wegs beträgt, welchen das Reaktionsgemisch nach der Zufuhr von Blausäure und Aceton vor Eintritt in eine Eintrittsöffnung des Reaktionsgemischs in mindestens eine Vorrichtung zum Vermischen des Reaktionsgemischs zurücklegt.

4. Verfahren nach einem der vorstehenden Ansprüche, **dadurch gekennzeichnet, dass** die Verweildauer des Katalysators im Reaktionsgemisch zwischen Zufuhr und Vermischung in mindestens einer Vorrichtung zum Vermischen des Reaktionsgemischs höchstens 10 s beträgt.

5. Verfahren nach einem der vorstehenden Ansprüche, **dadurch gekennzeichnet, dass** die Zufuhr des Katalysators kontinuierlich oder intermittierend erfolgt.

6. Verfahren nach einem der vorstehenden Ansprüche, **dadurch gekennzeichnet, dass** die Temperatur des Reaktionsgemischs im Schlaufenreaktor 11 bis 70 °C beträgt.

7. Verfahren nach einem der vorstehenden Ansprüche, **dadurch gekennzeichnet, dass** als Katalysator eine anorganische oder eine organische Base eingesetzt wird.

8. Verfahren nach einem der vorstehenden Ansprüche, **dadurch gekennzeichnet, dass** als Katalysator ein organisches Amin eingesetzt wird.

9. Verfahren nach einem der vorstehenden Ansprüche, **dadurch gekennzeichnet, dass** dem Schlaufenreaktor kontinuierlich oder intermittierend Reaktionsprodukt entzogen wird, wobei das Reaktionsprodukt nach Verlassen des Schlaufenreaktors in einem oder mehreren Wärmetauschern auf eine Temperatur von 0 bis 10 °C gekühlt wird.

10. Verfahren nach Anspruch 9, **dadurch gekennzeichnet, dass** das gekühlte Reaktionsprodukt mit einer Säure versetzt wird.

11. Verfahren nach Anspruch 10, **dadurch gekennzeichnet, dass** das Reaktionsprodukt mit Schwefelsäure versetzt wird.

12. Verfahren nach einem der Ansprüche 10 oder 11, **dadurch gekennzeichnet, dass** Schwefelsäure mit einen Wassergehalt von 0,1 bis 8 Gew.-% eingesetzt wird.

13. Verfahren nach einem der vorstehenden Ansprüche, **dadurch gekennzeichnet, dass** dem Schlaufenreaktor an mindestens einer Stelle eine Probenmenge des Reaktionsgemischs entnommen wird und die Probenmenge spektroskopisch untersucht wird.

14. Verfahren nach Anspruch 13, **dadurch gekennzeichnet, dass** die Probennahme kontinuierlich oder intermittierend erfolgt und die Probe dem Schlaufenreaktor nach der Messung wieder zugeführt wird.

15. Verfahren nach einem der vorstehenden Ansprüche, **dadurch gekennzeichnet, dass** die Reaktion bei einem pH-Wert von 6,5 bis 8 durchgeführt wird.

16. Verfahren zur Herstellung von Acetoncyanhydrin, bei dem ein Reaktionsgemisch enthaltend Aceton und Blausäure in Gegenwart eines Katalysators in einem Schlaufenreaktor unter Bildung von Acetoncyanhydrin als Reaktionsprodukt zur Reaktion gebracht werden und bei dem der Schlaufenreaktor mindestens eine Vorrichtung zum Kühlen des Reaktionsgemischs, mindestens eine Pumpe, mindestens eine Vorrichtung zum Vermischen des Reaktionsgemischs und jeweils mindestens eine Zufuhröffnung zum Zuführen von Blausäure, Aceton und Katalysator aufweist, wobei dem Schlaufenreaktor kontinuierlich oder intermittierend Reaktionsprodukt entzogen wird, das Reaktionsprodukt nach Verlassen des Schlaufenreaktors in einem oder mehreren Wärmetauschern auf eine Temperatur von 0 bis 10 °C gekühlt und mit Schwefelsäure mit einen Wassergehalt von 0,1 bis 8 Gew.-% versetzt wird und die Zufuhr des Katalysators stromabwärts von der Zufuhr von Aceton und Blausäure erfolgt.

17. Verfahren nach Anspruch 16, **dadurch gekennzeichnet, dass** die Zufuhr des Katalysators an einer Stelle des Schlaufenreaktors erfolgt, die näher an einer Eintrittsöffnung des Reaktionsgemischs in mindestens eine Vorrichtung zum Vermischen des Reaktionsgemischs liegt als an einer Zufuhröffnung zum Zuführen von Blausäure oder einer Zufuhröffnung zum Zuführen von Aceton oder beiden Zufuhröffnungen.

18. Verfahren nach einem der Ansprüche 16 bis 17, **dadurch gekennzeichnet, dass** die Zufuhr des Katalysators an einer Stelle des Schlaufenreaktors erfolgt, an welcher der Weg des Reaktionsgemischs vor Eintritt in eine Eintrittsöffnung des Reaktionsgemischs in mindestens eine Vorrichtung zum Vermischen des Reaktionsgemischs höchstens 30 % des Weges beträgt, welchen das Reaktionsgemisch nach der Zufuhr von Blausäure und Aceton vor Eintritt in eine Eintrittsöffnung des Reaktionsgemischs in mindestens eine Vorrichtung zum Vermischen des Reaktionsgemischs zurücklegt.

19. Verfahren nach einem der Ansprüche 16 bis 18, **dadurch gekennzeichnet, dass** die Verweildauer des Katalysators im Reaktionsgemisch zwischen Zufuhr und Vermischung in mindestens einer Vorrichtung zum Vermischen des Reaktionsgemischs höchstens 10 s beträgt.

20. Verfahren nach einem der Ansprüche 16 bis 19, **dadurch gekennzeichnet, dass** die Zufuhr des Katalysators kontinuierlich oder intermittierend erfolgt.

21. Verfahren nach einem der Ansprüche 16 bis 20, **dadurch gekennzeichnet, dass** die Temperatur des Reaktionsgemischs im Schlaufenreaktor 11 bis 70 °C beträgt.

22. Verfahren nach einem der Ansprüche 16 bis 21, **dadurch gekennzeichnet, dass** als Katalysator eine anorganische oder eine organische Base eingesetzt wird.

23. Verfahren nach einem der Ansprüche 16 bis 22, **dadurch gekennzeichnet, dass** als Katalysator ein organisches Amin eingesetzt wird.

24. Verfahren nach einem der Ansprüche 16 bis 23, **dadurch gekennzeichnet, dass** dem Schlaufenreaktor an mindestens einer Stelle eine Probenmenge des Reaktionsgemischs entnommen wird und die Probenmenge spektroskopisch untersucht wird.

25. Verfahren nach Anspruch 24, **dadurch gekennzeichnet, dass** die Probennahme kontinuierlich oder intermittierend erfolgt und die Probe dem Schlaufenreaktor nach der Messung wieder zugeführt wird.

26. Verfahren nach einem der Ansprüche 16 bis 25, **dadurch gekennzeichnet, dass** die Reaktion bei einem pH-Wert von 6,5 bis 8 durchgeführt wird.

## Claims

1. Process for preparing acetone cyanohydrin, in which a reaction mixture comprising acetone and hydrocyanic acid is reacted in the presence of a catalyst in a loop reactor to form acetone cyanohydrin as the reaction product, the loop reactor having at least one apparatus for cooling the reaction mixture, at least one pump, at least one apparatus for mixing the reaction mixture and at least one feed orifice for feeding hydrocyanic acid, acetone and catalyst, the catalyst being fed in downstream of the feed of acetone and hydrocyanic acid.

2. Process according to Claim 1, **characterized in that** the catalyst is fed in at a point in the loop reactor which is closer to an entry orifice of the reaction mixture into at least one apparatus for mixing the reaction mixture than to a feed orifice for feeding in hydrocyanic acid or a feed orifice for feeding in acetone or both feed orifices.

3. Process according to one of the preceding claims, **characterized in that** the catalyst is fed in at a point in the loop reactor at which the path length of the reaction mixture before entry into an entry orifice of the reaction mixture into at least one apparatus for mixing the reaction mixture is at most 30% of the path length that the reaction mixture travels after the feeding of hydrocyanic acid and acetone before entry into an entry orifice of the reaction mixture into at least one apparatus for mixing the reaction mixture.

4. Process according to one of the preceding claims, **characterized in that** the residence time of the catalyst in the reaction mixture between feed and mixing in at least one apparatus for mixing the reaction mixture is at most 10 s.

5. Process according to one of the preceding claims, **characterized in that** the catalyst is fed in continuously or intermittently.

6. Process according to one of the preceding claims, **characterized in that** the temperature of the reaction mixture in the loop reactor is 11 to 70°C.

7. Process according to one of the preceding claims, **characterized in that** the catalyst used is an inorganic or an organic base.

8. Process according to one of the preceding claims, **characterized in that** the catalyst used is an organic amine.

9. Process according to one of the preceding claims, **characterized in that** reaction product is withdrawn continuously or intermittently from the loop reactor, the reaction product being cooled to a temperature of 0 to 10°C in one or more heat exchangers after leaving the loop reactor.

10. Process according to Claim 9, **characterized in that** the cooled reaction product is admixed with an acid.

11. Process according to Claim 10, **characterized in that** the reaction product is admixed with sulphuric acid.

12. Process according to one of Claims 10 or 11, **characterized in that** sulphuric acid with a water content of 0.1 to 8% by weight is used.

13. Process according to one of the preceding claims, **characterized in that** a sample of the reaction mixture is withdrawn from the loop reactor at at least one point and the sample is analysed spectroscopically.

14. Process according to Claim 13, **characterized in that** the sampling is effected continuously or intermittently and the sample is fed back to the loop reactor after the analysis.

15. Process according to one of the preceding claims, **characterized in that** the reaction is performed at a pH of 6.5 to 8.

16. Process for preparing acetone cyanohydrin, in which a reaction mixture comprising acetone and hydrocyanic acid is reacted in the presence of a catalyst in a loop reactor to form acetone cyanohydrin as the reaction product, and in which the loop reactor has at least one apparatus for cooling the reaction mixture, at least one pump, at least one apparatus for mixing the reaction mixture and in each case at least one feed orifice for feeding hydrocyanic acid, acetone and catalyst, by continuously or intermittently withdrawing reaction product from the loop reactor, and cooling the reaction product, after leaving the loop reactor, to a temperature of 0 to 10°C in one or more heat exchangers and admixing it with sulphuric acid with a water content of 0.1 to 8% by weight and feeding in the catalyst downstream from the feed of acetone and hydrocyanic acid.

17. Process according to Claim 16, **characterized in that** the catalyst is fed in at a point in the loop reactor which is closer to an entry orifice of the reaction mixture in at least one apparatus for mixing the reaction mixture than to a feed orifice for feeding in hydrocyanic acid or a feed orifice for feeding in acetone or both feed orifices.

18. Process according to one of Claims 16 to 17, **characterized in that** the catalyst is fed in at a point in the loop reactor at which the path length of the reaction mixture before entry into an entry orifice of the reaction mixture into at least one apparatus for mixing the reaction mixture is at most 30% of the path length that the reaction mixture travels after the feeding of hydrocyanic acid and acetone before entry into an entry orifice of the reaction mixture into at least one apparatus for mixing the reaction mixture.

19. Process according to one of Claims 16 to 18, **characterized in that** the residence time of the catalyst in the reaction mixture between feed and mixing in at least one apparatus for mixing the reaction mixture is at most 10 s.

20. Process according to one of Claims 16 to 19, **characterized in that** the catalyst is fed in continuously or intermittently.

21. Process according to one of Claims 16 to 20, **characterized in that** the temperature of the reaction mixture in the loop reactor is 11 to 70°C.

22. Process according to one of Claims 16 to 21, **characterized in that** the catalyst used is an inorganic base or an organic base.

23. Process according to one of Claims 16 to 22, **characterized in that** the catalyst used is an organic amine.

24. Process according to one of Claims 16 to 23, **characterized in that** a sample of the reaction mixture is withdrawn from the loop reactor at at least one point and the sample is analysed spectroscopically.

25. Process according to Claim 24, **characterized in that** the sampling is effected continuously or intermittently and the sample is fed back to the loop reactor after the analysis.

26. Process according to one of Claims 16 to 25, **characterized in that** the reaction is performed at a pH of 6.5 to 8.

## Revendications

1. Procédé de fabrication de cyanhydrine d'acétone, selon lequel un mélange réactionnel contenant de l'acétone et de l'acide cyanhydrique est mis en réaction en présence d'un catalyseur dans un réacteur à boucle avec formation de cyanhydrine d'acétone en tant que produit de réaction, le réacteur à boucle comprenant au moins un dispositif pour le refroidissement du mélange réactionnel, au moins une pompe, au moins un dispositif pour le mélange du mélange réactionnel et à chaque fois au moins une ouverture d'alimentation pour l'alimentation d'acide cyanhydrique, d'acétone et de catalyseur, l'alimentation du catalyseur ayant lieu en aval de l'alimentation d'acétone et d'acide cyanhydrique.

2. Procédé selon la revendication 1, **caractérisé en ce que** l'alimentation du catalyseur a lieu à un emplacement du réacteur à boucle qui est plus proche d'une ouverture d'entrée du mélange réactionnel dans au moins un dispositif pour le mélange du mélange réactionnel que d'une ouverture d'alimentation pour l'alimentation d'acide cyanhydrique ou d'une ouverture d'alimentation pour l'alimentation d'acétone ou des deux ouvertures d'alimentation.

3. Procédé selon l'une quelconque des revendications précédentes, caractérisé ce que l'alimentation du catalyseur a lieu à un emplacement du réacteur à boucle auquel le parcours du mélange réactionnel avant l'entrée dans une ouverture d'entrée du mélange réactionnel dans au moins un dispositif pour le mélange du mélange réactionnel est d'au plus 30 % du parcours parcouru par le mélange réactionnel après l'alimentation de l'acide cyanhydrique et de l'acétone avant l'entrée dans une ouverture d'entrée du mélange réactionnel dans au moins un dispositif pour le mélange du mélange réactionnel.

4. Procédé selon l'une quelconque des revendications précédentes, **caractérisé en ce que** la durée de séjour du catalyseur dans le mélange réactionnel entre l'alimentation et le mélange dans au moins un dispositif pour le mélange du mélange réactionnel est d'au plus 10 s.

5. Procédé selon l'une quelconque des revendications précédentes, **caractérisé en ce que** l'alimentation du catalyseur a lieu de manière continue ou intermittente.

6. Procédé selon l'une quelconque des revendications précédentes, **caractérisé en ce que** la température du mélange réactionnel dans le réacteur à boucle est de 11 à 70 °C.

7. Procédé selon l'une quelconque des revendications précédentes, **caractérisé en ce qu'**une base inorganique ou organique est utilisée en tant que catalyseur.

8. Procédé selon l'une quelconque des revendications précédentes, **caractérisé en ce qu'**une amine organique est utilisée en tant que catalyseur.

9. Procédé selon l'une quelconque des revendications précédentes, **caractérisé en ce que** le produit de réaction est soutiré du réacteur à boucle de manière continue ou intermittente, le produit de réaction étant refroidi après la sortie du réacteur à boucle dans un ou plusieurs échangeurs de chaleur à une température de 0 à 10 °C.

10. Procédé selon la revendication 9, **caractérisé en ce que** le produit de réaction refroidi est mélangé avec un acide.

11. Procédé selon la revendication 10, **caractérisé en ce que** le produit de réaction est mélangé avec de l'acide sulfurique.

12. Procédé selon l'une quelconque des revendications 10 ou 11, **caractérisé en ce qu'**un acide sulfurique ayant une teneur en eau de 0,1 à 8 % en poids est utilisé.

13. Procédé selon l'une quelconque des revendications précédentes, **caractérisé en ce qu'**un échantillon du mélange réactionnel est soutiré du réacteur à boucle à au moins un emplacement et l'échantillon est analysé par spectroscopie.

14. Procédé selon la revendication 13, **caractérisé en ce que** l'échantillonnage a lieu de manière continue ou intermittente et l'échantillon est réintroduit dans le réacteur à boucle après la mesure.

15. Procédé selon l'une quelconque des revendications précédentes, **caractérisé en ce que** la réaction est réalisée à un pH de 6,5 à 8.

16. Procédé de fabrication de cyanhydrine d'acétone, selon lequel un mélange réactionnel contenant de l'acétone et de l'acide cyanhydrique est mis en réaction en présence d'un catalyseur dans un réacteur à boucle avec formation de cyanhydrine d'acétone en tant que produit de réaction, et selon lequel le réacteur à boucle comprend au moins un dispositif pour le refroidissement du mélange réactionnel, au moins une pompe, au moins un dispositif pour le mélange du mélange réactionnel et à chaque fois au moins une ouverture d'alimentation pour l'alimentation d'acide cyanhydrique, d'acétone et de catalyseur, le produit de réaction étant soutiré du réacteur à boucle de manière continue ou intermittente, le produit de réaction étant refroidi après la sortie du réacteur à boucle dans un ou plusieurs échangeurs de chaleur à une température de 0 à 10 °C, et mélangé avec de l'acide sulfurique ayant une teneur en eau de 0,1 à 8 % en poids, et l'alimentation du catalyseur ayant lieu en aval de l'alimentation d'acétone et d'acide cyanhydrique.

17. Procédé selon la revendication 16, **caractérisé en ce que** l'alimentation du catalyseur a lieu à un emplacement du réacteur à boucle qui est plus proche d'une ouverture d'entrée du mélange réactionnel dans au moins un dispositif pour le mélange du mélange réactionnel que d'une ouverture d'alimentation pour l'alimentation d'acide cyanhydrique ou d'une ouverture d'alimentation pour l'alimentation d'acétone ou des deux ouvertures d'alimentation.

18. Procédé selon l'une quelconque des revendications 16 à 17, **caractérisé en ce que** l'alimentation du catalyseur a lieu à un emplacement du réacteur à boucle auquel le parcours du mélange réactionnel avant l'entrée dans une ouverture d'entrée du mélange réactionnel dans au moins un dispositif pour le mélange du mélange réactionnel est d'au plus 30 % du parcours parcouru par le mélange réactionnel après l'alimentation de l'acide cyanhydrique et de l'acétone avant l'entrée dans une ouverture d'entrée du mélange réactionnel dans au moins un dispositif pour le mélange du mélange réactionnel.

19. Procédé selon l'une quelconque des revendications 16 à 18, **caractérisé en ce que** la durée de séjour du catalyseur dans le mélange réactionnel entre l'alimentation et le mélange dans au moins un dispositif pour le mélange du mélange réactionnel est d'au plus 10 s.

20. Procédé selon l'une quelconque des revendications 16 à 19, **caractérisé en ce que** l'alimentation du catalyseur a lieu de manière continue ou intermittente.

21. Procédé selon l'une quelconque des revendications 16 à 20, **caractérisé en ce que** la température du mélange réactionnel dans le réacteur à boucle est de 11 à 70 °C.

22. Procédé selon l'une quelconque des revendications 16 à 21, **caractérisé en ce qu'**une base inorganique ou organique est utilisée en tant que catalyseur.

23. Procédé selon l'une quelconque des revendications 16 à 22, **caractérisé en ce qu'**une amine organique est utilisée en tant que catalyseur.

24. Procédé selon l'une quelconque des revendications 16 à 23, **caractérisé en ce qu'**un échantillon du mélange réactionnel est soutiré du réacteur à boucle à au moins un emplacement et l'échantillon est analysé par spectroscopie.

25. Procédé selon la revendication 24, **caractérisé en ce que** l'échantillonnage a lieu de manière continue ou intermittente et l'échantillon est réintroduit dans le réacteur à boucle après la mesure.

26. Procédé selon l'une quelconque des revendications 16 à 25, **caractérisé en ce que** la réaction est réalisée à un pH de 6,5 à 8.
